# EUROPÄISCHE PATENTSCHRIFT

(11) **EP 1 426 372 B1**
(45) Veröffentlichungstag und Bekanntmachung des Hinweises auf die Patenterteilung: **11.01.2006**
(21) Anmeldenummer: 03026103.6
(22) Anmeldetag: 13.11.2003
(51) Int. Cl.: C07D 413/04, C07D 413/14, A61K 31/353, A61K 31/4245, A61P 35/00

(54) **2-Oxadiazolchromonderivate**
2-Oxadiazolechromone derivatives
Dérivés de 2-oxadiazolechromone

(30) Priorität: 02.12.2002 DE 10256182
(43) Veröffentlichungstag der Anmeldung: 09.06.2004
(73) Patentinhaber: Merck Patent GmbH, 64293 Darmstadt (DE)
(72) Erfinder: Mujica-Fernaud, Teresa, Dr., 64291 Darmstadt (DE); Buchholz, Herwig, Dr., 60599 Frankfurt (DE); Carola, Christophe, Dr., 63225 Langen (DE); Rautenberg, Wilfried, Dr., 64354 Reinheim (DE); Sirrenberg, Christian, Dr., 64289 Darmstadt (DE)

(56) Entgegenhaltungen:
- EP-A- 0 739 892
- EP-A- 0 758 649
- WO-A-01/16097
- WO-A-02/100826
- DE-A- 2 361 216
- DE-A- 3 834 204
- GB-A- 1 447 480
- BARRIO J R: "Compounds related to 2,4-dichlorophenoxyacetic acid and its derivatives." JOURNAL OF MEDICINAL CHEMISTRY. UNITED STATES MAR 1968, Bd. 11, Nr. 2, März 1968 (1968-03), Seiten 374-375, XP002274191 ISSN: 0022-2623

## Beschreibung

Der Erfindung lag die Aufgabe zugrunde, Verbindungen mit wertvollen Eigenschaften aufzufinden, insbesondere solche, die zur Herstellung von Arzneimitteln verwendet werden können.

Die vorliegende Erfindung betrifft Verbindungen, die die Signaltransduktion der Tyrosinkinase hemmen, regulieren und/oder modulieren, Zusammensetzungen, die diese Verbindungen enthalten, sowie Verfahren zu ihrer Verwendung zur Behandlung von tyrosinkinasebedingten Krankheiten und Leiden wie Krebs, Tumorwachstum, Arteriosklerose, altersbedingte Makula-Degeneration, diabetische Retinopathie, Entzündungserkrankungen und dergleichen bei Säugetieren.
Bei den Tyrosinkinasen handelt es sich um eine Klasse von Enyzmen, die die Übertragung des endständigen Phosphats des Adenosintriphosphats auf Tyrosinreste bei Proteinsubstraten katalysieren. Man nimmt an, dass den Tyrosinkinasen bei verschiedenen Zellfunktionen über die Substratphosphorylierung eine wesentliche Rolle bei der Signaltransduktion zukommt. Obwohl die genaue Mechanismen der Signaltransduktion noch unklar sind, wurde gezeigt, dass die Tyrosinkinasen wichtige Faktoren bei der Zellproliferation, der Karzinogenese und der Zelldifferenzierung darstellen.
Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen.

Die Rezeptor-Tyrosinkinasen bestehen aus einer Vielzahl von Transmembranrezeptoren mit unterschiedlicher biologischer Wirksamkeit. So wurden ungefähr 20 verschiedene Unterfamilien von Rezeptor-Tyrosinkinasen identifiziert. Eine Tyrosinkinase-Unterfamilie, die die Bezeichnung HER-Unterfamilie trägt, besteht aus EGFR, HER2, HER3 und HER4. Zu den Liganden dieser Rezeptor-Unterfamilie zählen der Epithel-Wachstumsfaktor, TGF-α, Amphiregulin, HB-EGF, Betacellulin und Heregulin. Die Insulin-Unterfamilie, zu der INS-R, IGF-IR und IR-R zählen, stellt eine weitere Unterfamilie dieser Rezeptor-Tyrosinkinasen dar. Die PDGF-Unterfamilie beinhaltet den PDGF-α- and -β-Rezeptor, CSFIR, c-kit und FLK-II. Außerdem gibt es die FLK-Familie, die aus dem Kinaseinsertdomänenrezeptor (KDR), der fötalen Leberkinase-1 (FLK-1), der fötalen Leberkinase-4 (FLK-4) und der fms-Tyrosinkinase-1 (flt-1) besteht. Die PDGF- und FLK-Familie werden üblicherweise aufgrund der zwischen den beiden Gruppen bestehenden Ähnlichkeiten gemeinsam diskutiert. Für eine genaue Diskussion der Rezeptor-Tyrosinkinasen siehe die Arbeit von Plowman et al., *DN &* P 7(6):334-339, 1994.
Die zytosolischen Tyrosinkinasen bestehen ebenfalls aus einer Vielzahl von Unterfamilien, darunter Src, Frk, Btk, Csk, Abi, Zap70, Fes/Fps, Fak, Jak, Ack, and LIMK. Jede dieser Unterfamilien ist weiter in verschiedene Rezeptoren unterteilt. So stellt zum Beispiel die Src-Unterfamilie eine der größten Unterfamilien dar. Sie beinhaltet Src, Yes, Fyn, Lyn, Lck, Blk, Hck, Fgr und Yrk. Die Src-Enzymunterfamilie wurde mit der Onkogenese in Verbindung gebracht. Für eine genauere Diskussion der zytosolischen Tyrosinkinasen, siehe die Arbeit von Bolen *Oncogene,* 8:2025-2031 (1993).
Sowohl die Rezeptor-Tyrosinkinasen als auch die zytosolischen Tyrosinkinasen sind an Signalübertragungswegen der Zelle, die zu verschiedenen Leidenszuständen führen, darunter Krebs, Schuppenflechte und Hyperimmunreaktionen, beteiligt.

Es wurde vorgeschlagen, dass verschiedene Rezeptor-Tyrosinkinasen sowie die an sie bindenden Wachstumsfaktoren eine Rolle bei den Angiogenese spielen, obwohl einige die Angiogenese indirekt fördern könnten (Mustonen und Alitalo, *J*. *Cell Biol*. 129:895-898, 1995). Eine dieser Rezeptor-Tyrosinkinasen ist die fötale Leberkinase 1, auch FLK-1 genannt. Das menschliche Analog der FLK-1 ist der kinase-insertdomänenhaltige Rezeptor KDR, der auch unter der Bezeichnung Gefäßendothelzellenwachstumsfaktorrezeptor 2 bzw. VEGFR-2 bekannt ist, da er VEGF hochaffin bindet. Schließlich wurde die Maus-Version dieses Rezeptors auch ebenfalls NYK genannt (Oelrichs et al., *Oncogene* 8(1):11-15, 1993). VEGF und KDR stellen ein Ligand-Rezeptor-Paar dar, das eine wesentliche Rolle bei der Proliferation der Gefäßendothelzellen und der Bildung und Sprossung der Blutgefäße, die als Vaskulogenese bzw. Angiogenese bezeichnet werden, spielt.
Die Angiogenese ist durch eine übermäßig starke Aktivität des Gefäßendothelwachstumsfaktors (VEGF) gekennzeichnet. Der VEGF besteht eigentlich aus einer Familie von Liganden (Klagsburn und D'Amore, *Cytokine & Growth Factor Reviews* 7:259-270, 1996). Der VEGF bindet den hochaffinen transmembranösen Tyrosinkinaserezepzor KDR und die verwandte fms-Tyrosinkinase-1, auch unter der Bezeichnung Flt-1 oder Gefäßendotheizellenwachstumsfaktorrezeptor 1 (VEGFR-1) bekannt. Aus Zellkultur- und Gen- Knockout-Versuchen geht hervor, dass jeder Rezeptor zu unterschiedlichen Aspekten der Angiogenese beiträgt. Der KDR führt die mitogene Funktion des VEGF herbei, während Flt-1 nichtmitogene Funktionen, wie diejenigen, die mit der Zelladhäsion in Zusammenhang stehen, zu modulieren scheint. Eine Hemmung des KDR moduliert daher das Niveau der mitogenen VEGF-Aktivität. Tatsächlich wurde gezeigt, dass das Tumorwachstum von der antiangiogenen Wirkung der VEGF-Rezeptor-Antagonisten beeinflusst wird (Kim et al., Nature 362, S. 841- 844, 1993).
Feste Tumore könne daher mit Tyrosinhemmern behandelt werden, da diese Tumore für die Bildung der zur Unterstützung ihres Wachstums erforderlichen Blutgefäße auf Angiogenese angewiesen sind. Zu diesen festen Tumoren zählen die Monozytenleukämie, Hirn-, .Urogenital-, Lymphsystem-, Magen-, Kehlkopf- und Lungenkarzinom, darunter Lungenadenokarzinom und kleinzelliges Lungenkarzinom. Zu weiteren Beispielen zählen Karzinome, bei denen eine Überexpression oder Aktivierung von Raf-aktivierenden Onkogenen (z.B. K-ras, erb-B) beobachtet wird. Zu diesen Karzinomen zählen Bauchspeicheldrüsen- und Brustkarzinom. Hemmstoffe dieser Tyrosinkinasen eignen sich daher zur Vorbeugung und Behandlung von proliferativen Krankheiten, die durch diese Enzyme bedingt sind.
Die angiogene Aktivität des VEGF ist nicht auf Tumore beschränkt. Der VEGF ist für die bei diabetischer Retinopathie in bzw. in der Nähe der Retina produzierte angiogene Aktivität verantwortlich. Dieses Gefäßwachstum in der Retina führt zu geschwächter Sehkraft und schließlich Erblindung. Die VEGF-mRNA- und -protein-Spiegel im Auge werden durch Leiden wie Netzhautvenenokklusion beim Primaten sowie verringertem pO₂-Spiegel bei der Maus, die zu Gefäßneubildung führen, erhöht. Intraokular injizierte monoklonale Anti-VEGF-Antikörper, oder VEGF-Rezeptor-Immunkonjugate, hemmen sowohl im Primaten- als auch im Nagetiermodell die Gefäßneubildung im Auge. Unabhängig vom Grund der Induktion des VEGF bei der diabetischen Retinopathie des Menschen, eignet sich die Hemmung des Augen-VEGF zur Behandlung dieser Krankheit.
Die VEGF-Expression ist auch in hypoxischen Regionen von tierischen und menschlichen Tumoren neben Nekrosezonen stark erhöht. Der VEGF wird außerdem durch die Expression der Onkogene ras, raf, src und p53-Mutante (die alle bei der Bekämpfung von Krebs von Bedeutung sind) hinaufreguliert. Monoklonale Anti-VEGF-Antikörper hemmen bei der Nacktmaus das Wachstum menschlicher Tumore. Obwohl die gleichen Tumorzellen in Kultur weiterhin VEGF exprimieren, verringern die Antikörper ihre Zellteilungsrate nicht. So wirkt der aus Tumoren stammende VEGF nicht als autokriner mitogener Faktor. Der VEGF trägt daher in vivo dadurch zum Tumorwachstum bei, dass er durch seine parakrine Gefäßendothelzellen-Chemotaxis- und -Mitogeneseaktivität die Angiogenese fördert. Diese monoklonalen Antikörper hemmen auch das Wachstum von typischerweise weniger stark vaskularisierten Human-Kolonkarzinomen bei thymuslosen Mäusen und verringern die Anzahl der aus inokulierten Zellen entstehenden Tumore.
Die Expression eines VEGF-bindenden Konstrukts von Flk-1, FIt-1, dem zur Entfernung der zytoplasmatischen Tyrosinkinasedomänen, jedoch unter Beibehaltung eines Membranankers, verkürzten Maus-KDR-Rezeptorhomologs, in Viren stoppt praktisch das Wachstum eines transplantierbaren Glioblastoms bei der Maus, vermutlich aufgrund des dominant-negativen Mechanismus der Heterodimerbildung mit transmembranösen Endothelzellen-VEGF-Rezeptoren. Embryostammzellen, die in der Nacktmaus üblicherweise in Form von festen Tumoren wachsen, bilden bei Knock-out aller beider VEGF-Allele keine nachweisbaren Tumore. Aus diesen Daten gemeinsam geht die Rolle des VEGF beim Wachstum fester Tumore hervor. Die Hemmung von von KDR bzw. Flt-1 ist an der pathologischen Angiogenese beteiligt, und diese Rezeptoren eignen sich zur Behandlung von Krankheiten, bei denen Angiogenese einen Teil der Gesamtpathologie, z.B. Entzündung, diabetische Retina-Vaskularisierung sowie verschiedene Formen von Krebs, darstellt, da bekannt ist, dass das Tumorwachstum angiogeneseabhängig ist (Weidner et al., N. Engl. J. Med., 324, S. 1-8, 1991).

Bei Angiopoieten 1 (Ang1), einem Liganden für die endothelspezifische Rezeptor-Tyrosinkinase TIE-2, handelt es sich um einen neuen angiogenen Faktor (Davis et al, Cell, 1996, 87:1161-1169; Partanen et al, Mol. Cell Biol., 12:1698-1707 (1992); US-Patent Nr. 5,521,073; 5,879,672; 5,877,020; und 6,030,831). Das Akronym TIE steht für "Tyrosinkinase mit lg- und EGF-Homologiedomänen". TIE wird zur Identifizierung einer Klasse von Rezeptor-Tyrosinkinasen verwendet, die ausschließlich in Gefäßendothelzellen und frühen hämopoietischen Zellen exprimiert werden. TIE-Rezeptorkinasen sind typischerweise durch das Vorhandensein einer EGF-ähnlichen Domäne und einer Immunglobulin (IG)-ähnlichen Domäne charakterisiert, die aus extrazellulären Faltungseinheiten, die durch Disulfidbrückenbindungen zwischen den Ketten stabilisiert sind, besteht (Partanen et al Curr. Topics Microbiol. Immunol., 1999, 237:159-172). Im Gegensatz zu VEGF, der seine Funktion während der frühen Stadien in der Gefäßentwicklung ausübt, wirken Ang1 und sein Rezeptor TIE-2 während der späteren Stadien in der Gefäßentwicklung, d.h. während der Gefäßumbildung (Umbildung bezieht sich auf die Bildung eines Gefäßlumens) und Reifung (Yancopoulos et al, Cell, 1998, 93:661-664; Peters, K.G., Circ. Res., 1998, 83(3):342-3; Suri et al, Cell 87, 1171-1180 (1996)).

Demzufolge würde man erwarten, daß eine Hemmung von TIE-2 die Umbildung und Reifung eines durch Angiogenese initiierten neuen Gefäßsystems und dadurch den Angiogeneseprozeß unterbrechen sollte. Weiterhin würde eine Hemmung an der Kinasedomäne-Bindungsstelle von VEGFR-2 die Phosphorylierung von Tyrosinresten blockieren und dazu dienen, die Initiation der Angiogenese zu unterbrechen. Daher darf man annehmen, daß die Hemmung von TIE-2 und/oder VEGFR-2 die Tumorangiogenese verhindern und dazu dienen sollte, das Tumorwachstum zu verlangsamen oder vollständig zu beseitigen. Dementsprechend könnte man eine Behandlung von Krebs und anderen mit unangemessener Angiogenese einhergehenden Erkrankungen bereitstellen.

Die vorliegende Erfindung richtet sich auf Verfahren zur Regulation, Modulation oder Hemmung der TIE-2 zur Vorbeugung und/oder Behandlung von Erkrankungen im Zusammenhang mit unregulierter oder gestörter TIE-2-Aktivität. Insbesondere lassen sich die erfindungsgemäßen. Verbindungen auch bei der Behandlung gewisser Krebsformen einsetzen.

Weiterhin können die erfindungsgemäßen Verbindungen verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Die vorliegende Erfindung betrifft weiterhin die Verbindungen als Inhibitoren von Raf-Kinasen.
Protein-Phosphorylierung ist ein fundamentaler Prozess für die Regulation von Zellfunktionen. Die koordinierte Wirkung von sowohl Proteinkinasen als auch Phosphatasen kontrolliert die Phosphorylierungsgrade und folglich die Aktivität spezifischer Zielproteine. Eine der vorherrschenden Rollen der Protein-Phosphorylierung ist bei der Signaltransduktion, wenn extrazelluläre Signale amplifiziert und durch eine Kaskade von Protein-Phosphorylierungs- und Dephosphorylierungsereignissen, z. B. im p21^{ras}/raf-Weg propagiert werden.

Das p21^{ras}-Gen wurde als ein Onkogen der Harvey- und Kirsten-Ratten-Sarkom-Viren (H-Ras bzw. K-Ras) entdeckt. Beim Menschen wurden charakteristische Mutationen im zellulären Ras-Gen (c-Ras) mit vielen verschiedenen Krebstypen in Verbindung gebracht. Von diesen mutanten Allelen, die Ras konstitutiv aktiv machen, wurde gezeigt, dass sie Zellen, wie zum Beispiel die murine Zelllinie NIH 3T3, in Kultur transformieren.

Das p21^{ras}-Onkogen ist ein wichtiger beitragender Faktor bei der Entwicklung und Progression humaner solider Karzinome und ist bei 30 % aller humaner Karzinome mutiert (Bolton et al. (1994) Ann. Rep. Med. Chem., 29, 165-74; Bos. (1989) Cancer Res., 49, 4682-9). In seiner normalen, nicht mutierten Form ist das Ras-Protein ein Schlüsselelement der Signaltransduktionskaskade, die durch Wachstumsfaktor-Rezeptoren in fast allen Geweben gesteuert wird (Avruch et al. (1994) Trends Biochem. Sci., 19, 279-83).

Biochemisch ist Ras ein Guanin-Nukleotid-bindendes Protein, und das Zyklieren zwischen einer GTP-gebundenen aktivierten und einer GDPgebundenen ruhenden Form wird von Ras-endogener GTPase-Aktivität und anderen Regulatorproteinen strikt kontrolliert. Das Ras-Genprodukt bindet an Guanintriphosphat (GTP) und Guanindiphosphat (GDP) und hydrolysiert GTP zu GDP. Ras ist im GTP-gebundenen Zustand aktiv. In den Ras-Mutanten in Krebszellen ist die endogene GTPase-Aktivität abgeschwächt, und folglich gibt das Protein konstitutive Wachstumssignale an "Downstream"-Effektoren, wie zum Beispiel an das Enzym Raf-Kinase ab. Dies führt zum krebsartigen Wachstum der Zellen, die diese Mutanten tragen (Magnuson et al. (1994) Semin. Cancer Biol., 5, 247-53). Das Ras-Proto-Onkogen benötigt ein funktionell intaktes C-Raf-1-Proto-Onkogen, um in höheren Eukaryoten durch Rezeptor- und Nicht-Rezeptor-Tyrosin-Kinasen initiierte Wachstums- und Differenzierungssignale zu transduzieren.

Aktiviertes Ras ist für die Aktivierung des C-Raf-1-Proto-Onkogens notwendig, die biochemischen Schritte, durch die Ras die Raf-1-Protein-(Ser/Thr)-Kinase aktiviert, sind jedoch inzwischen gut charakterisiert. Es wurde gezeigt, dass das Inhibieren des Effekts von aktivem Ras durch Inhibition des Raf-Kinase-Signalwegs mittels Verabreichung von deaktivierenden Antikörpern gegen Raf-Kinase oder mittels Koexpression dominanter negativer Raf-Kinase oder dominanter negativer MEK (MAPKK), dem Substrat der Raf-Kinase, zur Reversion transformierter Zellen zum normalen Wachstumsphänotyp führt, siehe: Daum et al. (1994) Trends Biochem. Sci., 19, 474-80; Fridman et al. (1994) J Biol. Chem., 269, 30105-8. Kolch et al. (1991) Nature, 349, 426-28) und zur Besprechung Weinstein-Oppenheimer et al. Pharm. & Therap. (2000), 88, 229-279.

Auf ähnliche Weise wurde die Inhibition von Raf-Kinase (durch Antisense-Oligodesoxynukleotide) in vitro und in vivo mit der Inhibition des Wachstums einer Reihe verschiedener humaner Tumortypen in Beziehung gebracht (Monia et al., Nat. Med. 1996, 2, 668-75).

Raf-Serin- und Threonin-spezifische Protein-Kinasen sind cytosolische Enzyme, die das Zellwachstum in einer Reihe verschiedener Zellsysteme stimulieren (Rapp, U.R., et al. (1988) in The Oncogene Handbook; T. Curran, E.P. Reddy und A. Skalka (Hrsg.) Elsevier Science Publishers; Niederlande, S. 213-253; Rapp, U.R., et al. (1988) Cold Spring Harbor Sym. Quant. Biol. 53:173-184; Rapp, U.R., et al. (1990) Inv Curr. Top. Microbiol. Immunol. Potter und Melchers (Hrsg.), Berlin, Springer-Verlag 166:129-139).

### Drei Isozyme wurden charakterisiert:

C-Raf (Raf-1) (Bonner, T.I., et al. (1986) Nucleic Acids Res. 14:1009-1015). A-Raf (Beck, T.W., et al. (1987) Nucleic Acids Res. 15:595-609), und B-Raf (Qkawa, S., et al. (1998) Mol. Cell. Biol. 8:2651-2654; Sithanandam, G. et al. (1990) Oncogene:1775). Diese Enzyme untercheiden sich durch ihre Expression in verschiedenen Geweben. Raf-1 wird in allen Organen und in allen Zelllinien, die untersucht wurden, exprimiert, und A- und B-Raf werden in Urogenital- bzw. Eürngeweben exprimiert (Storm, S.M. (1990) Oncogene 5:345-351).

Raf-Gene sind Proto-Onkogene: Sie können die maligne Transformation von Zellen initiieren, wenn sie in spezifisch veränderten Formen exprimiert werden. Genetische Veränderungen, die zu onkogener Aktivierung führen, erzeugen eine konstitutiv aktive Proteinkinase durch Entfernung oder Interferenz mit einer N-terminalen negativen Regulatordomäne des Proteins (Heidecker, G., et al. (1990) Mol. Cell. Biol. 10:2503-2512; Rapp, U.R., et al. (1987) in Oncogenes and Cancer; S. A. Aaronson, J. Bishop, T. Sugimura, M. Terada, K. Toyoshima und P. K. Vogt (Hrsg.) Japan Scientific Press, Tokyo). Mikroinjektion in NIH 3T3-Zellen von onkogen aktivierten, aber nicht Wildtyp-Versionen des mit Expressionsvektoren von Escherichia coli präparierten Raf-Proteins führt zu morphologischer Transormation und stimuliert die DNA-Synthese (Rapp, U.R., et al. (1987) in Oncogenes and Cancer; S. A. Aaronson, J. Bishop, T. Sugimura, M. Terada, K. Toyoshima, und P. K. Vogt (Hrsg.) Japan Scientific Press, Tokyo; Smith, M. R., et al. (1990) Mol. Cell. Biol. 10:3828-3833).

Folglich ist aktiviertes Raf-1 ein intrazellulärer Aktivator des Zellwachstums. Raf-1-Protein-Serin-Kinase ist ein Kandidat für den "Downstream"-Effektor der Mitogen-Signaltransduktion, da Raf-Onkogene dem Wachstumsarrest begegnen, der aus einer Blockade zellulärer Ras-Aktivität aufgrund einer zellulären Mutation (Ras-revertante Zellen) oder Mikroinjektion von Anti-Ras-Antikörpern resultiert (Rapp, U.R., et al. (1988) in The Oncogene Handbook, T. Curran, E.P. Reddy und A. Skalka (Hrsg.), Elsevier Science Publishers; Niederlande, S. 213-253; Smith, M. R., et al. (1986) Nature (London) 320:540-543).

Die C-Raf-Funktion ist für die Transformation durch eine Reihe verschiedener Membran-gebundener Onkogene und für die Wachstumsstimutation durch in Sera enthaltene Mitogene erforderlich (Smith, M.R., et al. (1986) Nature (London) 320:540-543). Raf-1-Protein-Serin-Kinase-Aktivität wird durch Mitogene über die Phosphorylierung reguliert (Morrison, D.K., et al. (1989) Cell 58:648-657), welche auch die subzelluläre Verteilung bewirkt (Olah, Z., et al. (1991) Exp. Brain Res. 84:403; Rapp, U.R., et al. (1988) Cold Spring Harbor Sym. Quant. Biol. 53:173-184. Zu Raf-1-aktivierenden Wachstumsfaktoren zählen der aus Thrombozyten stammende Wachstumsfaktor (PDGF) (Morrison, D.K., et al. (1988) Proc. Natl. Acad. Sci. USA 85:8855-8859), der Kolonien-stimulierende Faktor (Baccarini, M., et al. (1990) EMBO J. 9:3649-3657), Insulin (Blackshear, P.J., et al. (1990) J. Biol. Chem. 265:12115-12118), der epidermale Wachstumsfaktor (EGF) (Morrison, R.K., et al. (1988) Proc. Natl. Acad. Sci. USA 85:8855-8859), Interleukin-2 (Turner, B.C., et al. (1991) Proc. Natl. Acad. Sci. USA 88:1227) und Interleukin-3 und der Granulozyten-Makrophagen-Kolonien-stimulierende Faktor (Carroll, M.P., et al. (1990) J. Biol. Chem. 265:19812-19817).

Nach der Mitogen-Behandlung von Zellen transloziert die transient aktivierte Raf-1-Protein-Serin-Kinase in den perinukleären Bereich und den Nukleus (Olah, Z., et al. (1991) Exp. Brain Res. 84:403; Rapp, U.R., et al. (1988) Cold Spring Habor Sym. Quant. Biol. 53:173-184). Zellen, die aktiviertes Raf enthalten, sind in ihrem Genexpressionsmuster verändert (Heidecker, G., et al. (1989) in Genes and signal transduction in multistage carcinogenesis, N. Colburn (Hrsg.), Marcel Dekker, Inc., New York, S. 339-374) und Raf-oncogenes activate transcription from Ap-1/PEA3-dependent promotors in transient transfection assays (Jamal, S., et al. (1990) Science 344:463-466; Kaibuchi, K., et al. (1989) J. Biol. Chem. 264:20855-20858; Wasylyk, C., et al. (1989) Mol. Cell. Biol. 9:2247-2250).

Es gibt mindestens zwei unabhängige Wege für die Raf-1-Aktivierung durch extrazelluläre Mitogene: Einen, der Proteinkinase C (KC) beinhaltet, und einen zweiten, der durch Protein-Tyrosin-Kinasen initiiert wird (Blackshear, P.J., et al. (1990) J. Biol. Chem. 265:12131-12134; Kovacina, K.S., et al. (1990) J. Biol. Chem. 265:12115-12118; Morrison, D.K., et al. (1988) Proc. Natl. Acad. Sci. USA 85:8855-8859; Siegel, J.N., et al. (1990) J. Biol. Chem. 265:18472-18480; Turner, B.C., et al. (1991) Proc. Natl. Acad. Sci. USA 88:1227). In jedem Fall beinhaltet die Aktivierung Raf-1-Protein-Phosphorylierung. Raf-1-Phosphorylierung kann eine Folge einer Kinase-Kaskade sein, die durch Autophosphorylierung amplifiziert wird, oder kann vollkommen durch Autophosphorylierung hervorgerufen werden, die durch Bindung eines vermutlichen Aktivierungsliganden an die Raf-1-Regulatordomäne, analog zur PKC-Aktivierung durch Diacylglycerol initiiert wird (Nishizuka, Y. (1986) Science 233:305-312).

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Proteinkinase PKB (auch als AKT und RAC-PK bekannt) ist ein Mitglied der AKT/PKB-Familie der Serin-/Threonin-Kinasen, und es wurde gezeigt, dass sie an einer diversen Reihe von Signalwegen bei der humanen Malignität beteiligt ist (Nicholson et al., Cell. Signal., 2002, 14, 381-395). PKB, wie auch andere Mitglieder der AKT/PKB-Familie, ist im Cytosol nicht stimulierter Zellen lokalisiert und transloziert nach Stimulation an die Zellmembran. PKB-Translokation kann durch mehrere Liganden, einschließlich des aus Thrombozyten stammenden Wachstumsfaktors, epidermalen Wachstumfaktors, basischen Fibroblasten-Wachstumsfaktors, Zellstress, wie zum Beispiel Hitzeschock und Hyperosmolarität und auch Insulin, aktiviert werden (Bos, Trends Biochem. Sci., 1995, 20, 441-442), und andere Studien haben gezeigt, dass diese Aktivierung über PI3-Kinase abläuft, die Wortmannin-empfindlich ist (Franke et al., Science, 1997, 275, 665-668). Sobald PKB an der Plasmamembran lokalisiert ist, wurde gezeigt, dass sie mehrere Funktionen in der Zelle vermittelt, einschließlich Apoptose, der metabolischen Effekte von Insulin, Induktion von Differenzierung und/oder Proliferation, Proteinsynthese und Stressantworten (Alessi und Cohen, Curr. Opin. Genet. Dev., 1998, 8, 55-62; Downward, Curr. Opin. Cell Biol., 1998, 10, 262-267).

PKB wurde 1991 von drei Gruppen unabhängig geklont (Bellacosa et al., Science, 1991, 254, 274-277; Coffer und Woodgett, Eur. J. Biochem., 1991, 201, 475-481; Jones et al., Cell Regul., 1991, 2, 1001- 1009), ihr Zusammenhang mit dem primären humanen Magenkarzinom wurde jedoch bereits 1987 erkannt (Staal et al., Proc. Natl. Acad. Sci. U S A, 1987, 84, 5034-5037). Sequenzierung von PKBα ließ in den Kinasedomänen Homologie zu den PKA- (ca. 68 %) und PKC-Isozymen (ca. 73 %) erkennen (Jones et al., Proc. Natl. Acad. Sci. U.S.A., 1991, 88, 4171-5), eine Tatsache, die zu ihrer Umbenennung in PKB führte. Es gibt drei zelluläre PKB-Isoformen und zwei Splice-Varianten (PKBα, β, γ, β₁, γ₁; Brazil et al. Trends in Bio Sci, 2001, 26, 657-663). Es wurde gefunden, dass PKBα bei Magenadenokarzinomen und einer Brustkrebs-Zeillinie amplifiziert oder überexprimiert wird (Staal et al., Proc. Natl. Acad. Sci. U.S.A., 1987, 84, 5034-7; Jones et al., Cell Regul., 1991, 2, 1001-9). PKBβ wird bei 3 % des Brust- (Bellacosa et al., Int. J. Cancer, 1995 64, 280-5), 12 % des Pankreas- (Cheng et al., Proc. Natl. Acad. Sci. U.S.A., 1996, 93, 3636-41) und 15 % des Eierstockkrebses amplifiziert oder überexprimiert (Bellacosa et al., Int. J. Cancer, 1995, 64, 280-5; Cheng et al., Proc. Natl. Acad. Sci. U.S.A., 1992, 89, 9267-71).

PKBγ wird bei Estrogenrezeptor-defizientem Brustkrebs und bei Androgenunabhängigen Prostata-Zelllinien überexprimiert (Nakatani et al., J. Biol. Chem. 1999, 274, 21528-32).

Es wurde vorgeschlagen, dass PKB ein an der chromosomalen Umordnung an der Chromosomenbande 14q32 beteiligtes Gen ist. Von diesem Locus ist bekannt, dass er bei humanen T-Zellmalignitäten, wie zum Beispiel bei prolymphozytischen Leukämien und Leukämien gemischter Abstammung im Kindersalter der Umordnung unterliegt (Staal et al., Genomics, 1988, 2, 96-98).

PKB spielt auch eine Rolle bei der Verhinderung des "programmierten Zelltodes" oder der Apoptose durch inhibitorische Phosphorylierung von ASK-1, Bad, Caspase9 und FKHR (Übersicht siehe Nicholson et al., Cell Signaling 2001, 14, 281-395). Es wurde nachgewiesen, dass PKB ein Überlebenssignal (Übersicht siehe Lawlor et al., J. of Cell Science 2001, 114, 2903-2910) für Zellen liefert, um sie vor einer Anzahl von Agenzien, einschließlich UV-Strahlung (Dudek et al., Science, 1997, 275, 661-665), Entzug von IGF1 aus neuronalen Zellen, Ablösung von der extrazellulären Matrix, Stress und Hitzeschock zu schützen (Alessi und Cohen, Curr. Opin. Genet. Dev., 1998, 8, 55-62).

Die dual-spezifische Phosphatase PTEN (Phosphatase and Tensin homologue deleted on Chromosome Ten [Phosphatase und Tensin homolog deletiert an Chromosom zehn]) erhöht den Ptdlns(3, 4, 5)P₃-Spiegel in der Zelle durch Dephosphorylierung von Ptdlns(3, 4, 5)P₃. Ptdlns(3, 4, 5)P₃ bindet an die PH-Domäne (Pleckstrin-Homologie- Domäne) von PKB. Diese Bindung stellt einen wesentlichen Schritt für die Membran-Translokation und Aktivierung von PKB dar. PTEN ist ein in einer großen Fraktion von Glioblastom- und Melanom-Zelllinien, fortgeschrittenen Prostatakarzinomen und endometrialen Karzinomen mutiertes Tumor-Suppressor-Gen. Es ist darüber hinaus bei >80 % der Patienten mit erblichen Leiden, wie zum Beispiel Cowden-Syndrom, Lhermitte-Duclos-Syndrom und Bannayan-Zonana-Syndrom deletiert. Die Patienten weisen mehrere ähnliche Merkmale auf, einschließlich multipler gutartiger Tumoren (Harmatomen) und einer erhöhten Anfälligkeit für Brust- und Schilddrüsenmaügnitäten (Di Cristofano et al. Cell, 2000, 100, 387-390).

Von heterozygoten PTEN^{+/-}-Mäusen (heterozygote PTEN^{-/-}-Mäuse sind nicht lebensfähig) hergeleitete Zelllinien weisen erhöhte Ptdlns(3, 4, 5)P₃₋Spiegel auf, die mit erhöhter PKB-Aktivität, mit einer gleichzeitig verminderten Sensitivität gegen Apoptose einhergehen (Di Christofano et al. Nat. Genet. 1998, 19, 348-355; Stambolic et al., Cell, 1998, 95, 29-39, Myers et al., Proc. Natl. Acad. Si. U.S.A., 1998, 96 13513-13518).

PKB ist auch zur Promotion der Zellzyklus-Progression durch Inhibition des p21-Zellzyklus-Inhibitors fähig (Zhou et al.; Nat. Cell Biol., 2002,3, 245-252).

Diese Befunde könnten die Überexperession von PKB erklären, die in Krebszellen beobachtet wird, die präferenzielles Überleben und Proliferation der Karzinome durch Verhindern der normalen Progression zur Apoptose ermöglicht.
Zur Zeit gibt es keine bekannten Therapeutika, welche die PKB-Aktivität wirksam inhibieren. Folglich besteht noch immer ein seit langem wahrgenommener Bedarf an zusätzlichen Mitteln, die als Chemotherapeutika zur wirksamen Inhibition der PKB-Funktion zur Aktivierung von proapoptotischen Proteinen bei allen Krebsarten fähig sind.

Die Identifikation von kleinen Verbindungen, die die Signaltransduktion der Tyrosinkinasen, insbesondere TIE-2 und/oder Raf-Kinasen spezifisch hemmen, regulieren und/oder modulieren, ist daher wünschenswert und ein Ziel der vorliegenden Erfindung.

Es wurde gefunden, daß die Verbindungen der Formel I und ihre Salze bei guter Verträglichkeit sehr wertvolle pharmakologische Eigenschaften besitzen. Insbesondere zeigen sie inhibierende Eigenschaften der Tyrosinkinase und insbesondere von TIE-2.

Die erfindungsgemäßen Verbindungen eignen sich darüberhinaus als Nahrungsmittelzusatzstoffe, zur Behandlung von Krankheiten und/oder Fehlfunktionen, die durch oxidative Stressbedingungen charakterisiert sind, sowie als Nahrungsmittelzusatzstoffe, ferner in kosmetischen Formulierungen als Sonnenschutzmittel.

### ZUSAMMENFASSUNG DER ERFINDUNG

Die Erfindung betrifft Verbindungen der Formel I 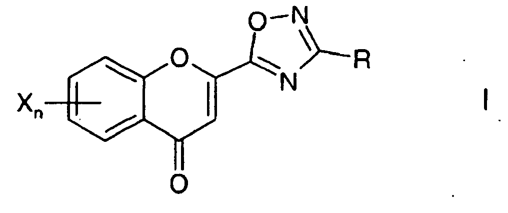
worin
- R: A, 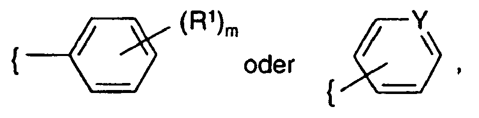
- X: H, -OH, -OA, Phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO oder SO₂NH₂,
zwei Reste X zusammen auch Methylendioxy oder Ethylendioxy,
- R¹: H, A, -OH, -OA oder Hal,
zwei Reste R¹ zusammen auch Methylendioxy oder Ethylendioxy,
- Y: CH oder N,
- Ar: unsubstituiertes oder ein-, zwei- oder dreifach durch A substituiertes Phenyl,

- A: unverzweigtes oder verzweigtes Alkyl mit 1-10 C-Atomen, wobei 1-7 H-Atome durch F ersetzt sein können,
- Hal: F, Cl, Br oder I,
- n: 1, 2, 3 oder 4,
- m: 1, 2, 3, 4 oder 5
bedeuten,
sowie ihre pharmazeutisch verwendbaren Derivate, Solvate und Stereoisomere, einschließlich deren Mischungen in allen Verhältnissen.

Gegenstand der Erfindung sind die Verbindungen der Formel I und ihre Salze sowie ein Verfahren zur Herstellung von Verbindungen der Formel I nach den Ansprüchen 1-4 sowie ihrer pharmazeutisch verwendbaren Derivate, Solvate und Stereoisomere, dadurch gekennzeichnet, daß man
a) zunächst eine Verbindung der Formel II 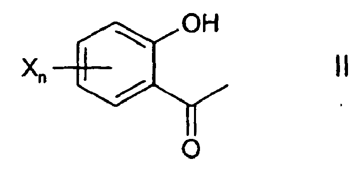 worin
X und n die in Anspruch 1 angegebene Bedeutung haben, mit einer Verbindung der Formel III 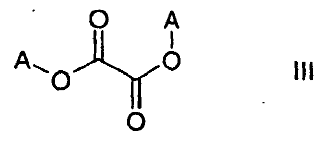
worin A Alkyl mit 1, 2, 3, 4, 5 oder 6 C-Atomen bedeutet, zu einer Verbindung der Formel IV 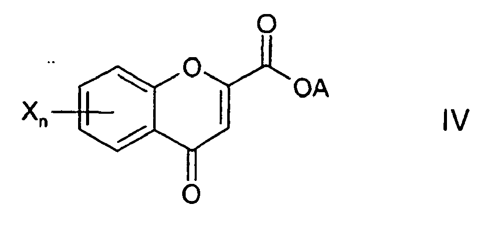
worin X und n die in Anspruch 1 angegebene Bedeutung haben und A Alkyl mit 1, 2, 3, 4, 5 oder 6 C-Atomen bedeutet,
umsetzt,
b) anschließend den Ester IV zur Carbonsäure V hydrolysiert 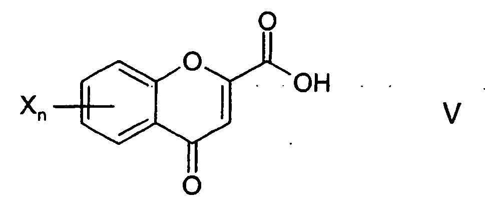
   worin X und n die in Anspruch 1 angegebene Bedeutung haben,
c) anschließend die Carbonsäure V in das entsprechende Säurechlorid VI 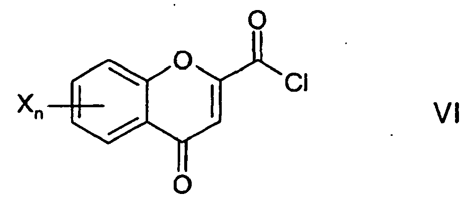
   worin X und n die in Anspruch 1 angegebene Bedeutung haben, überführt,
   und dann
   entweder die Verbindung der Formel VI mit einer Verbindung der Formel VII 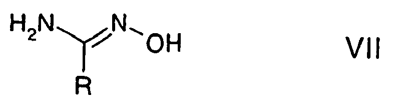
   worin R die in Anspruch 1 angegebene Bedeutung hat,
   zu einer Verbindung der Formel 1 umsetzt,
   oder die Verbindung der Formel V mit einer Verbindung der Formel VII,
   worin R die in Anspruch 1 angegebene Bedeutung hat,
   in einer zweistufigen Eintopf-Reaktion zu einer Verbindung der Formel I umsetzt,
   und/oder
d) eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen. ,

Für alle Reste, die mehrfach auftreten, wie z.B. A, gilt, daß deren Bedeutungen unabhängig voneinander sind.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

OA bedeutet Alkoxy und ist vorzugsweise z.B. Methoxy, Ethoxy, Propoxy, Isopropoxy, Butoxy, Trifluormethoxy oder Cyclopentoxy.

-COA (Acyl) bedeutet vorzugsweise Acetyl, Propionyl, ferner auch Butyryl, Pentanoyl, Hexanoyl oder z.B. Benzoyl.
Hal bedeutet vorzugsweise F, Cl oder Br, aber auch I

X bedeutet vorzugsweise -OH, -OA, Phenoxy oder -O-CO-A, insbesondere OH oder OA, ganz besonders bevorzugt OH.

R bedeutet vorzugsweise A oder 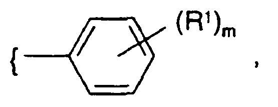 besonders bevorzugt Alkylphenyl, ganz besonders bevorzugt tert.-Butylphenyl.

Die Verbindungen der Formel I können in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Id ausgedrückt werden, die der Formel I entsprechen und worin die nicht näher bezeichneten Reste die bei der Formel I angegebene Bedeutung haben, worin jedoch
- in la: X H, -OH, -OA, zwei Reste X zusammen auch Methylendioxy oder Ethylendioxy, bedeutet;
in lb
- R: A oder 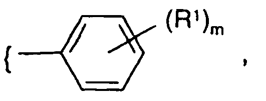
- X: H, -OH, -OA,
zwei Reste X zusammen auch Methylendioxy oder Ethylendioxy, bedeuten;
in Ic
- R: A, 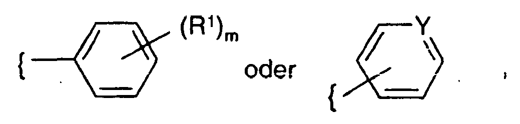
- X: H, -OH oder -OA,
- Y: N,
- R¹: A,
- A: unverzweigtes oder verzweigtes Alkyl mit 1-6 C-Atomen,
- n: 1, 2, 3 oder 4,
- m: 1, 2, 3 oder 4,
bedeuten;
in Id
- R: 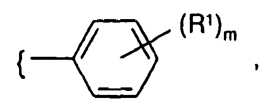
- X: H, -OH oder -OA,
- R¹: A,
- A: unverzweigtes oder verzweigtes Alkyl mit 1-6 C-Atomen,
- n: 1, 2, 3 oder 4,
- m: 1, 2, 3 oder 4,
bedeuten, sowie ihre pharmazeutisch verwendbaren Derivate, Solvate und Stereoisomere, einschließlich deren Mischungen in allen Verhältnissen.

Gegenstand der Erfindung sind insbesondere die nachstehenden Verbindungen der Formel I
6-Hydroxy-2-[3-(4-tert.-butylphenyl)-[1,2,4]-oxadiazol-5-yl]-chromon,
7-Hydroxy-2-[3-(4-tert.-butylphenyl)-[1,2,4]-oxadiazol-5-yl]-chromon,
sowie ihre pharmazeutisch verwendbaren Derivate, Solvate und Stereoisomere, einschließlich deren Mischungen in allen Verhältnissen.

Die Verbindungen der Formel 1 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II und III sind in der Regel bekannt. Sind sie neu, so können sie aber nach an sich bekannten Methoden hergestellt werden.

Verbindungen der Formel 1 können vorzugsweise erhalten werden, indem man Verbindungen der Formel II mit Verbindungen der Formel III zuerst zu Verbindungen der Formel IV umsetzt.

Die Umsetzung erfolgt nach Methoden, die dem Fachmann bekannt sind. Zunächst erfolgt Reaktion in einem geeigneten Alkohol in Gegenwart eines Alkali- oder Erdalkalialkoholats, z.B. in Ethanol/Natrium-ethanolat oder Methanol/Kalium-methanolat.

Grundsätzlich können auch die nachfolgenden inerten Lösungsmittel verwendet werden.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, lsopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Die Cyclisierung zur Verbindung der Formel IV erfolgt säurekatalysiert, durch Zugabe geeigneter Mineralsäuren, wie z.B. Salzsäure, Phosphorsäure oder Schwefelsäure.

Reaktionszeit und Temperatur sind vorzugsweise wie oben angegeben.

Die Ester der Formel IV können z.B. mit Essigsäure und/oder HCl oder mit NaOH oder KOH in Wasser, Wasser-THF oder Wasser-Dioxan bei Temperaturen zwischen 0 und 100° zu den Carbonsäuren der Formel V verseift werden.

Die Verbindungen der Formel V werden nach Standardmethoden z.B. mit Oxalylchlorid in einem inerten Lösungsmittel zu dem entsprechenden Säurechlorid der Formel VI umgesetzt.
Inertes Lösungsmittel, Reaktionszeit und Temperatur sind vorzugsweise wie oben angegeben.

Die Umsetzung der Verbindungen der Formel VI mit den Verbindungen der Formel VII zu den Verbindungen der Formel I erfolgt unter basenkatalysierten Bedingungen in einem inerten Lösungsmittel, wie sie dem Fachmann bekannt sind.
Inertes Lösungsmittel, Reaktionszeit und Temperatur sind vorzugsweise wie oben angegeben.
Als Basen eignen sich anorgansiche und organische Basen, wie z.B. Pyridin.

Die Umsetzung der Verbindungen der Formel V mit den Verbindungen der Formel VII zu den Verbindungen der Formel I erfolgt in einer zweistufigen Eintopf-Reaktion.
Zunächst wird die Verbindung der Formel V in ein geeignetes gemischtes Anhydrid überführt, z.B. mit Isobutylchloroformiat.

Die Reaktion erfolgt unter basenkatalysierten Bedingungen in einem inerten Lösungsmittel, wie sie dem Fachmann bekannt sind.
Inertes Lösungsmittel, Reaktionszeit und Temperatur sind vorzugsweise wie oben angegeben.
Als Basen eignen sich anorgansiche und organische Basen, wie z.B. Pyridin oder Triethylamin.
Anschließend erfolgt die Reaktion mit der Verbindung der Formel VII in einem inerten Lösungsmittel.
Inertes Lösungsmittel, Reaktionszeit und Temperatur sind vorzugsweise wie oben angegeben.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsultonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Gegenstand der Erfindung sind ferner Arzneimittel enthaltend mindestens eine Verbindung der Formel 1 und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

### VERWENDUNG

Einzelheiten

### I.

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung von Verbindungen gemäß einem oder mehreren der Ansprüche 1 bis 5 und/oder ihre physiologisch unbedenklichen Salze und Solvate zur Herstellung eines Arzneimittels zur Behandlung oder Vorbeugung von Krebs bei einem Säugetier, das einer derartigen Behandlung bedarf, wobei man bei diesem Verfahren dem Säugetier eine therapeutisch wirksame Menge einer Verbindung der Formel I verabreicht. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.
Ebensfalls umfasst ist die Verwendung von Verbindungen gemäß einem oder mehreren der Ansprüche 1 bis 5 und/oder ihre physiologisch unbedenklichen Salze und Solvate zur Herstellung eines Arzneimittels zur Behandlung oder Vorbeugung einer Krankheit, an der Angiogenese beteiligt ist, wobei man bei diesem Verfahren einem Säugetier, das einer derartigen Behandlung bedarf, eine therapeutisch wirksame Menge einer Verbindung der Formel I verabreicht. Eine derartige Krankheit, an der Angiogenese beteiligt ist, ist eine Augenkrankheit, wie Retina-Vaskularisierung, diabetische Retinopathie, altersbedingte Makula-Degeneration und dergleichen.

Die Verwendung von Verbindungen gemäß einem oder mehreren der Ansprüche 1 bis 5 und/oder ihre physiologisch unbedenklichen Salze und Solvate zur Herstellung eines Arzneimittels zur Behandlung oder Vorbeugung von Entzündungskrankheiten, wobei man bei diesem Verfahren einem Säugetier, das einer derartigen Behandlung bedarf, eine therapeutisch wirksame Menge einer Verbindung der Formel I verabreicht, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung von Verbindungen gemäß einem oder mehreren der Ansprüche 1 bis 5 und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer Verbindung der Formel 1 verabreicht. Die therapeutische Menge hängt von der jeweiligen Krankheit ab und kann vom Fachmann ohne allen großen Aufwand bestimmt werden.
Die vorliegende Erfindung umfasst auch die Verwendung von Verbindungen gemäß einem oder mehreren der Ansprüche 1 bis 5 und/oder ihre physiologisch unbedenklichen Salze und Solvate zur Herstellung eines Arzneimittels zur Behandlung oder Vorbeugung von Retina-Vaskularisierung, wobei man bei diesem Verfahren einem Säugetier, das einer derartigen Behandlung bedarf, eine therapeutisch wirksame Menge einer Verbindung der Formel I verabreicht. Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung. Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubüdung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die erfindungsgemäßen Verbindungen können an Patienten zur Behandlung von Krebs verabreicht werden. Die vorliegenden Verbindungen hemmen die Tumorangiogenese und beeinflussen so das Wachstum von Tumoren (J. Rak et al. *Cancer Research,* 55:4575-4580, 1995). Die angiogenesehemmenden Eigenschaften der vorliegenden Verbindungen eignen sich auch zur Behandlung bestimmter Formen von Blindheit, die mit Retina-Gefäßneubildung in Zusammenhang stehen. Die offenbarten Verbindungen eignen sich auch zur Behandlung bestimmter Knochen-Pathologien wie Osteosarkom, Osteoarthritis und Rachitis, die auch unter der Bezeichnung onkogene Osteomalazie bekannt ist (Hasegawa et al., Skeletal Radiol. 28, S.41-45,1999; Gerber et al., Nature Medicine, Bd. 5, Nr. 6, S.623-628, Juni 1999). Da der VEGF durch den in reifen Osteoklasten exprimierten KDR/Flk-1 direkt die osteoklastische Knochenresorption fördert (FEBS Let. 473:161-164 (2000); Endocrinology, 141:1667 (2000)), eignen sich die vorliegenden Verbindungen auch zur Behandlung und Vorbeugung von Leiden, die mit Knochenresorption in Zusammenhang stehen, wie Osteoporose und Morbus Paget.
Die beanspruchten Verbindungen können dadurch, dass sie zerebrale Ödeme, Gewebeschädigung und ischämiebedingte Reperfusionsverletzungen reduzieren, auch zur Verringerung oder Vorbeugung von Gewebeschäden, die nach zerebralen ischämischen Ereignissen wie Gehirnschlag auftreten, verwendet werden (*Drug News Perspect* 11:265-270 (1998); *J. Clin. Invest*. 104:1613-1620 (1999)).

Die erfindungsgemäßen Verbindungen können auch gemeinsam mit anderen gut bekannten Therapeutika, die aufgrund ihrer jeweiligen Eignung für das behandelte Leiden ausgewählt werden, verabreicht werden. So wären zum Beispiel bei Knochenleiden Kombinationen günstig, die antiresorptiv wirkende Bisphosphonate, wie Alendronat und Risedronat, Integrinblocker (wie sie weiter unten definiert werden), wie avβ3-Antagonisten, bei der Hormontherapie verwendetete konjugierte Östrogene wie Prempro®, Premarin® und Endometrion®; selektive Östrogenrezeptormodulatoren (SERMs) wie Raloxifen, Droloxifen, CP-336,156 (Pfizer) und Lasofoxifen, Kathepsin-K-Hemmer und ATP-Protonenpumpenhemmer enthalten.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186). "Östrogenrezeptormodutatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-(2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll. "Androgenrezeptormodulatoren" bezieht sich auf Verbindungen., die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diaminplatin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll. Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, lrinotecan, Rubitecan, 6-Ethoxypropionyl-3',4'-O-exo-benzylidenchartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1 H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethylamino)-ethyl)acridin-4-carboxarnid, 6-[[2-(Dimethylamino)ethyl]amino]-3-hydroxy-7H-indeno[2,1 -c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-mannoheptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehydthiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Vorzugsweise handelt es sich bei den Tyrosinkinasen um TIE-2. Besonders bevorzugt ist die Verwendung der erfindungsgemäßen Verbindungen zur Herstellung eines Arzneimittels zur Behandlung von Krankheiten, die durch Inhibierung von TIE-2 durch die Verbindungen nach Anspruch 1 beeinflußt werden.
Insbesondere bevorzugt ist die Verwendung zur Behandlung einer Krankheit, wobei die Krankheit ein fester Tumor ist.

Der feste Tumor ist vorzugsweise ausgewählt aus der Gruppe Gehirntumor, Tumor des Urogenitaltrakts, Tumor des lymphatischen Systems, Magentumor, Kehlkopftumor und Lungentumor.

Der feste Tumor ist weiterhin vorzugsweise ausgewählt aus der Gruppe Monozytenleukämie, Lungenadenokarzinom, kleinzellige Lungenkarzinome, Bauchspeicheldrüsenkrebs, Glioblastome und Brustkarzinom.

Es wurde weiterhin gefunden, daß die Verbindungen der Formel 1, die im Allgemeinen als Chromenon-Derivate beschrieben werden, Inhibitoren des Enzyms Raf-Kinase sind. Da das Enzym ein "Downstream"- Effektor von p21^{ras} ist, erweisen sich die Inhibitoren in pharmazeutischen Zusammensetzungen für die human- oder veterinärmedizinische Anwendung als nützlich, wenn Inhibition des Raf-Kinase-Weges, z. B. bei der Behandlung von Tumoren und/oder durch Raf-Kinase vermitteltem krebsartigen Zellwachstum, angezeigt ist. Die Verbindungen sind insbesondere nützlich bei der Behandlung solider Karzinome bei Mensch und Tier, z. B. von murinem Krebs, da die Progression dieser Krebse abhängig ist von der Ras-Protein-Signaltransduktionskaskade und deshalb auf die Behandlung durch Unterbrechung der Kaskade, d. h. durch Inhibition der Raf-Kinase, anspricht. Dementsprechend wird die Verbindung der Formel I oder ein pharmazeutisch unbedenkliches Salz davon für die Behandlung von Krankheiten verabreicht, die durch den Raf-Kinase-Weg vermittelt werden, besonders Krebs, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom), pathologische Angiogenese und metastatische Zellmigration. Die Verbindungen sind ferner nützlich bei der Behandlung der Komplementaktivierungsabhängigen chronischen Entzündung (Niculescu et al. (2002) Immunol. Res., 24:191-199) und durch HIV-1 (Human Immunodeficiency Virus Typ 1) induzierte Immunschwäche (Popik et al. (1998) J Virol, 72: 6406-6413).
Es wurde überraschend gefunden, dass erfindungsgemäße Verbindungen Derivate mit Signalwegen, besonders mit den hierin beschriebenen Signalwegen und bevorzugt dem Raf-Kinase-Signalweg interagieren können. Erfindungsgemäße Chromenon-Derivate zeigen bevorzugt eine vorteilhafte biologische Aktivität, die in auf Enzymen basierenden Assays, zum Beispiel Assays wie hierin beschrieben, leicht nachweisbar ist. In derartigen, auf Enzymen basierenden Assays zeigen und bewirken erfindungsgemäße Chromenon-Derivate bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Wie hierin besprochen, sind diese Signalwege für verschiedene Erkrankungen relevant. Dementsprechend sind Chromenon-Derivate nützlich bei der Prophylaxe und/oder Behandlung von Erkrankungen, die von den genannten Signalwegen durch Interaktion mit einem oder mehreren der genannten Signalwege abhängig sind.
Gegenstand der vorliegenden Erfindung sind deshalb erfindungsgemäße Verbindungen als Promotoren oder Inhibitoren, bevorzugt als Inhibitoren der hierin beschriebenen Signalwege. Bevorzugter Gegenstand der Erfindung sind deshalb erfindungsgemäße Verbindungen als Promotoren oder Inhibitoren, bevorzugt als Inhibitoren des Raf-Kinase-Weges. Ein bevorzugterer Gegenstand der Erfindung sind deshalb erfindungsgemäße Derivate als Promotoren oder Inhibitoren, bevorzugt als Inhibitoren der Raf-Kinase. Ein noch bevorzugterer Gegenstand der Erfindung sind erfindungsgemäße Verbindungen als Promotoren oder Inhibitoren, bevorzugt als Inhibitoren einer oder mehrerer Raf-Kinasen, ausgewählt aus der Gruppe bestehend aus A-Raf, B-Raf und C-Raf-1. Ein besonders bevorzugter Gegenstand der Erfindung sind erfindungsgemäße Verbindungen als Promotoren oder Inhibitoren, bevorzugt als Inhibitoren von C-Raf-1.

Ein weiterer Gegenstand der vorliegenden Erfindung ist die Verwendung einer oder mehrerer erfindungsgemäßer Verbindungen bei der Behandlung und/oder Prophylaxe von Erkrankungen, bevorzugt den hier beschriebenen Erkrankungen, die durch Raf-Kinasen veruracht, vermittelt und/oder propagiert werden und insbesondere Erkrankungen, die durch Raf-Kinasen ausgewählt aus der Gruppe, bestehend aus A-Raf, B-Raf and C-Raf-1 verursacht, vermittelt und/oder propagiert werden. Gewöhnlich werden die hier besprochenen Erkrankungen in zwei Gruppen eingeteilt, in hyperproliferative und nicht hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiteri und Immunschwächekrankheiten als nicht krebsartige Krankheiten angesehen, von denen Arthritis, Entzündung, immunologische Krankheiten, Autoimmunkrankheiten und Immunschwächekrankheiten gewöhnlich als nicht hyperproliferative Erkrankungen angesehen werden. In diesem Zusammenhang sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich als hyperproliferative Erkrankungen angesehen werden. Insbesondere krebsartiges Zellwachstum und insbesondere durch Raf-Kinase vermitteltes krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Als Vergleichsmessung wurde weiterhin gefunden, daß die Verbindungen der Formel I als schlechte PKB-Inhibitoren wirken. Diese Wirkung kann zum Beispiel durch ein Verfahren nachgewiesen werden, das von Alessi et al. EMBO L. 1996, 15, 6541-6551 beschrieben wird.

Es kann gezeigt werden, dass die erfindungsgemäßen Verbindungen in in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Die erfindungsgemäßen Verbindungen können an Säugetiere, vorzugsweise den Menschen, entweder allein oder vorzugsweise in Kombination mit pharmazeutisch unbedenklichen Trägern oder Streckmitteln, gegebenenfalls mit bekannten Hilfsstoffen wie Alaun, in einer pharmazeutischen Zusammensetzung wie in der pharmazeutischen Praxis üblich verabreicht werden. Die Verbindungen können oral oder parenteral verabreicht werden, darunter auf dem intravenösen, intramuskulären, intreperitonealen, subkutanen, rektalen und topischen Verabreichungsweg.
Bei der oralen Verwendung einer erfindungsgemäßen chemotherapeutischen Verbindung kann die gewählte Verbindung zum Beispiel in Form von Tabletten oder Kapseln oder als wässrige Lösung oder Suspension verabreicht werden. Bei Oraltabletten zählen zu den üblicherweise verwendeten Trägern Laktose und Maisstärke, und es werden üblicherweise Gleitmittel wie Magnesiumstearat zugegeben. Bei der oralen Verabreichung in Kapselform zählen Laktose und getrocknetete Maisstärke zu geeigneten Streckmitteln. Sind wässrige Suspensionen zur oralen Verwendung erforderlich, so wird der Wirkstoff mit Emulgatoren und Suspendiermitteln zusammengegeben. Gewünschtenfalls können bestimmte Süßstoffe und/oder Aromastoffe zugegeben werden. Für die intramuskuläre, intraperitoneale, subkutane und intravenöse Verwendung werden üblichwerweise sterile Lösungen des Wirkstoffs hergestellt, und der pH-Wert der Lösungen sollte auf geeignete Weise eingestellt und gepuffert werden. Bei der intravenösen Verwendung sollte die Gesamtkonzentration an gelösten Substanzen so eingestellt werden, dass das Präparat isotonisch wird.
Die genaue Dosis für den jeweiligen Patienten hängt jedoch von einer Reihe von Faktoren ab, beispielsweise von der Wirksamkeit der jeweiligen verwendeten Verbindungen, vom Alter, Körpergewicht, allgemeinen Gesundheitszustand, Geschlecht, von der Ernährung, von Verabreichungszeitpunkt und -weg, von der Ausscheidungsrate, dem Verabreichungstyp, der zu verabreichenden Arzneiform, der Medikamentenkombination und der Schwere der Krankheit, gegen die die Therapie eingesetzt wird. Die jeweilige therapeutisch wirksame Dosis für den jeweiligen Patienten kann leicht durch Routineversuche bestimmt werden, zum Beispiel durch den Arzt, der zu dieser therapeutischen Behandlung rät bzw. sie begleitet.

Die erfindungsgemäßen Substanzen werden in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des. Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche.

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., *Cancer Res.* 59:189-197; Xin et al:, J. *Biol. Chem.* 274:9116-9121; Sheu et al., *Anticancer Res.* 18:4435-4441; Ausprunk et al., *Dev. Biol.* 38:237-248; Gimbrone et al., J. *Natl. Cancer Inst*. 52:413-427; Nicosia et al., *In Vitro* 18:538- 549). VEGF-Rezeptorkinase-Assay
Die VEGF-Rezeptorkinaseaktivität wird durch Einbau von radioaktiv markiertem Phosphat in 4:1 Polyglutaminsäure/Tyrosin-Substrat (pEY) bestimmt. Das phosphorylierte pEY-Produkt wird auf einer Filtermembran festgehalten, und der Einbau des radioaktiv markierten Phosphats wird durch Szintillationszählung quantitativ bestimmt.

### MATERIALIEN

### VEGF-Rezeptorkinase

Die intrazelluläre-Tyrosinkinase-Domänen des menschlichen KDR (Terman, B. I. et al. Oncogene (1991) Bd. 6, S. 1677-1683.) und Flt-1 (Shibuya, M. et al. Oncogene (1990) Bd. 5, S. 519-524) wurden als Glutathion-S-transferase (GST)-Genfusionsproteine kloniert. Dies geschah durch Klonieren der Zytoplasma-Domäne der KDR-Kinase als leserastergerechte Fusion am Carboxy-Terminus des GST-Gens. Die löslichen rekombinanten GST-Kinasedomäne-Fusionsproteine wurden in *Spodoptera frugiperda* (Sf21) Insektenzellen (Invitrogen) unter Verwendung eines Baculovirus-Expressionsvektors (pAcG2T, Pharmingen) exprimiert.
Lysepuffer
50 mM Tris pH 7,4, 0,5 M NaCl, 5 mM DTT, 1 mM EDTA, 0,5% Triton X-100, 10% Glycerin, je 10 mg/ml Leupeptin, Pepstatin und Aprotinin sowie 1 mM Phenylmethylsulfonylfluorid (alle von Sigma).
Waschpuffer
50 mM Tris pH 7,4, 0,5 M NaC1, 5 mM DTT, 1 mM EDTA, 0.05% Triton X-100, 10% Glycerin, je 10 mg/ml Leupeptin, Pepstatin und Aprotinin sowie 1 mM Phenylmethylsulfonylfluorid.
Dialysepuffer
50 mM Tris pH 7,4, 0,5 M NaC1, 5 mM DTT, 1 mM EDTA, 0.05% Triton X-100, 50% Glycerin, je 10 mg/mi Leupeptin, Pepstatin und Aprotinin sowie 1 mM Phenylmethylsulfonylfluorid.
10x Reaktionspuffer
200 mM Tris, pH 7,4, 1,0 M NaCl, 50 mM MnCl₂, 10 mM DTT und 5 mg/ml Rinderserumalbumin [bovine serum albumin = BSA] (Sigma).
Enzymverdünnungspuffer
50 mM Tris, pH 7,4, 0,1 M NaCl, 1 mM DTT, 10% Glycerin, 100 mg/ml BSA.
10x Substrat
750 µg/ml Poly(glutaminsäure/Tyrosin; 4:1) (Sigma).
Stopp-Lösung
30% Trichloressigsäure, 0,2 M Natriumpyrophosphat (beide von Fisher). Waschlösung 15% Trichloressigsäure, 0,2 M Natriumpyrophosphat.
Filterplatten.
Millipore #MAFC NOB, GF/C 96-Well-Glasfaserplatte.

### Verfahren A - Proteinaufreinigung

1. Die Sf21-Zellen wurden mit dem rekombinanten Virus bei einer m.o.i. (Multiplizität der Infektion) von 5 Viruspartikeln/Zelle infiziert und 48 Stunden lang bei 27°C gezüchtet.
2. Alle Schritte wurden bei 4°C durchgeführt. Die infizierten Zellen wurden durch Zentrifugieren bei 1000xg geerntet und 30 Minuten bei 4°C mit 1/10 Volumen Lysepuffer lysiert und anschließend 1 Stunde lang bei 100.000×g zentrifugiert. Der Überstand wurde dann über eine mit Lysepuffer äquilibrierte Glutathion-Sepharose-Säure (Pharmacia) gegeben und mit 5 Volumina des gleichen Puffers und anschließend 5 Volumina Waschpuffer gewaschen. Das rekombinante GST-KDR-Protein wurde mit Waschpuffer/10 mM reduziertem Glutathion (Sigma) eluiert und gegen Dialysepuffer dialysiert.

### Verfahren B - VEGF-Rezeptorkinase-Assay

1. Assay mit 5 µl Hemmstoff oder Kontrolle in 50% DMSO versetzen.
2. Mit 35 µl Reaktionsmischung, die 5 µl 10x Reaktionspuffer, 5 µl 25 mM ATP/10 µCi[³³ P]ATP (Amersham) und 5 µl 10x Substrat enthält, versetzen.
3. Reaktion durch Zugabe von 10 µl KDR (25 nM) in Enzymverdünnungspuffer starten.
4. Mischen und 15 Minuten lang bei Raumtemperatur inkubieren.
5. Reaktion durch Zugabe von 50 µl Stopp-Lösung stoppen.
6. 15 Minuten lang bei 4°C inkubieren.
7. 90-µl-Aliquot auf Filterplatte überführen.
8. Absaugen und 3 Mal mit Waschlösung waschen.
9. 30 µl Szintillations-Cocktail zugeben, Platte verschließen und in einem Szintillatiöns-Zähler Typ Wallac Microbeta zählen.
Mitogenese-Assay an menschlichen Nabelschnurvenenendothelzellen Die Expression von VEGF-Rezeptoren, die mitogene Reaktionen auf den Wachstumsfaktor vermitteln, ist größtenteils auf Gefäßendothelzellen beschränkt. Kultivierte menschliche Nabelschnurvenenendothelzellen (HUVECs) proliferieren als Reaktion auf Behandlung mit VEGF und können als Assaysystem zur quantitativen Bestimmung der Auswirkungen von KDR-Kinasehemmern auf die Stimulation des VEGF verwendet werden. In dem beschriebenen Assay werden Einzelzellschichten von HUVECs im Ruhezustand 2 Stunden vor der Zugabe von VEGF oder "basic fibroblast growth factor" (bFGF) mit dem Konstituens oder der Testverbindung behandelt. Die mitogene Reaktion auf VEGF oder bFGF wird durch Messung des Einbaus von [³H]Thymidin in die Zell-DNA bestimmt.

### Materialien

### HUVECs

Als Primärkulturisolate tiefgefrorene HUVECs werden von Clonetics Corp bezogen. Die Zellen werden im Endothel-Wachstumsmedium (Endothelial Growth Medium = EGM; Clonetics) erhalten und in der 3. - 7. Passage für die Mitogenitätsassays verwendet.
Kulturplatten
NUNCLON 96-Well-Polystyrol-Gewebekulturplattten (NUNC #167008). Assay-Medium
Nach Dulbecco modifiziertes Eagle-Medium mit 1 g/ml Glucose (DMEM mit niedrigem Glucosegehalt; Mediatech) plus 10% (v/v) fötales Rinderserum (Clonetics).
Testverbindungen
Mit den Arbeitsstammlösungen der Testverbindungen wird mit 100% Dimethylsulfoxid (DMSO) solange eine Reihenverdünnung durchgeführt, bis ihre Konzentrationen um das 400-fache höher als die gewünschte Endkonzentration sind. Die letzten Verdünnungen (Konzentration 1 x) werden unmittelbar vor Zugabe zu den Zellen mit Assay-Medium hergestellt.
10x Wachstumsfaktoren
Lösungen des menschlichen VEGF 165 (500 ng/ml; R&D Systems) und bFGF (10 ng/ml; R&D Systems) werden mit Assay-Medium hergestellt. 10×[³H]-Thymidin [Methyl-³H]-Thymidin (20 Ci/mmol; Dupont-NEN) wird mit DMEM-Medium mit niedrigem Glucosegehalt auf 80 µCi/ml verdünnt.
Zellwaschmedium
Hank's balanced salt solution (Mediatech) mit 1 mg/ml Rinderserumalbumin (Boehringer-Mannheim).
Zell-Lyse-Lösung
1 N NaOH, 2% (w/v) Na₂CO₃.
Verfahren 1
In EGM gehaltene HUVEC-Einzelzellschichten werden durch Trypsinbehandlung geerntet und in einer Dichte von 4000 Zellen pro 100 µl Assay-Medium pro Näpfchen in 96-Well-Platten überimpft. Das Wachstum der Zellen wird 24 Stunden bei 37°C in einer 5% CO₂ enthaltenden feuchten Atmosphäre gestoppt.
Verfahren 2
Das Wachstumsstoppmedium wird durch 100 µl Assay-Medium ersetzt, das entweder das Konstituens (0,25% [v/v] DMSO) oder die erwünschte Endkonzentration der Testverbindung enthält. Alle Bestimmungen werden in dreifacher Wiederholung durchgeführt. Die Zellen werden dann 2 Stunden bei 37°C/5% CO₂ inkubiert, so dass die Testverbindungen in die Zellen eindringen können.
Verfahren 3
Nach 2-stündiger Vorbehandlung werden die Zellen durch Zugabe von 10 µl Assay-Medium, 10x VEGF-Lösung oder 10x bFGF-Lösung pro Näpfchen stimuliert. Die Zellen werden dann bei 37°C/5% CO₂ inkubiert. Verfahren 4
Nach 24 Stunden in Anwesenheit der Wachstumsfaktoren wird mit 10x [³H]-Thymidin (10 µl/well) versetzt.
Verfahren 5
Drei Tage nach dem Versetzen mit [³H]-Thymidin wird das Medium abgesaugt und die Zellen werden zweimal mit Zellwaschmedium gewaschen (400 µl/well, anschließend 200 µl/well). Die gewaschenen, adhärenten Zellen werden dann durch Zugabe von Zell-Lyse-Lösung (100 µl/well) und 30-minutiges Erwärmen auf 37°C solubilisiert. Die Zell-Lysate werden in 7-ml-Szintillationsrährchen aus Glas, die 150 µl Wasser enthalten, überführt. Man versetzt mit dem Szintillations-Cocktail (5 ml/Röhrchen), und die mit den Zellen assoziierte Radioaktivität wird flüssigkeitsszintillationsspektroskopisch bestimmt.
Gemäß diesen Assays stellen die Verbindungen der Formel 1 VEGF-Hemmer dar und eignen sich daher zur Hemmung der Angiogenese, wie bei der Behandlung von Augenkrankheiten, z.B. diabetischer Retinopathie, und zur Behandlung von Karzinomen, z.B. festen Tumoren. Die vorliegenden Verbindungen hemmen die VEGF-stimulierte Mitogenese von kultivierten menschlichen Gefäßendotheizellen mit HK50-Werten von 0,01-5,0 µM. Diese Verbindungen sind im Vergleich zu verwandten Tyrosinkinasen (z.B. FGFR1 sowie Src-Familie; zur Beziehung zwischen Src-Kinasen und VEGFR-Kinasen siehe Eliceiri et al., Molecular Cell, Bd. 4, S.915-924, Dezember 1999) auch selektiv.

### II.

Gegenstand der Erfindung ist weiterhin die Verwendung von Verbindungen gemäß einem oder mehreren der Ansprüche 1 bis 4 und/oder ihre physiologisch unbedenklichen Salze und Solvate zur Herstellung eines Arzneimittels zur Behandlung von Krankheiten und/oder Fehlfunktionen, die durch oxidative Stressbedingungen charakterisiert sind, bei einem Säugetier, das einer derartigen Behandlung bedarf, wobei man dem Säugetier, das einer derartigen Behandlung bedarf, eine therapeutisch wirksame Menge einer Verbindung nach Anspruch 1 oral verabreicht.

Bei den Krankheiten und/oder Fehlfunktionen handelt es sich insbesondere um Gedächtnisverlust und um neurodegenerative Erkrankungen handelt.
Die erfindungsgemäßen Verbindungen finden daher Verwendung zur Neuroprotektion.

Die Verwendung von Isoquercetin und Ascorbinsäure zu entsprechenden Zwecken ist in der WO 00/54754 beschrieben.

Gegenstand der Erfindung ist weiterhin die Verwendung der erfindungsgemäßen Verbindungen und/oder ihre physiologisch unbedenklichen Salze und Solvate als Nahrungsmittelzusatzstoffe.

Gegenstand der Erfindung ist weiterhin eine Zusammensetzung, enthaltend Ascorbinsäure, Ascorbat oder ein Ascorbinsäurederivät und mindestens eine erfindungsgemäße Verbindung und/oder ihr physiologisch unbedenkliches Salz und Solvat.

### III.

Die erfindungsgemäßen Verbindungen weisen als Flavonoidderivate antioxidative Eigenschaften auf.

Die Verwendung von Ectoinderivaten mit Antioxidationsmitteln zum Schutz von Stressproteinen der Haut in topischen Formulierungen ist z.B. in der WO 01/72263 beschrieben.

Gegenstand der Erfindung ist daher die Verwendung der erfindungsgemäßen Verbindungen und/oder ihre physiologisch unbedenklichen Salze und Solvate in kosmetischen Formulierungen, vorzugsweise in Form einer topischen Formulierung.

Gegenstand der Erfindung ist weiterhin die Verwendung der erfindungsgemäßen Verbindungen und/oder ihre physiologisch unbedenklichen Salze und Solvate zum Schutz der Streßproteine der Haut, vorzugsweise in Form einer topischen Formulierung.

Die Herstellung der topischen Zusammensetzung erfolgt, indem mindestens eine der erfindungsgemäß verwendeten Verbindungen, gegebenenfalls mit Hilfs- und/oder Trägerstoffen, in eine geeignete Formulierungsform gebracht werden. Die Hilfs- und Trägerstoffe stammen aus der Gruppe der Trägermittel, Konservierungsstoffe und anderer üblicher Hilfsstoffe.

Die topischen Zusammensetzungen auf der Grundlage mindestens einer erfindungsgemäß verwendeten Verbindung wird äußerlich auf der Haut oder den Hautadnexen angewendet.

Als Anwendungsform seien z.B. genannt, Lösungen, Suspensionen, Emulsionen, Pasten, Salben, Gele, Cremes, Lotionen, Puder, Seifen, tensidhaltige Reinigungspräparate, Öle und Sprays. Zusätzlich zu einer oder mehreren erfindungsgemäß verwendeten Verbindungen werden der Zusammensetzung beliebige übliche Trägerstoffe, Hilfsstoffe und gegebenenfalls weitere Wirkstoffe zugesetzt.

Bevorzugte Hilfsstoffe stammen aus der Gruppe der Konservierungsstoffe, Antioxidantien, Stabilisatoren, Lösungsvermittler, Vitamine, Färbemittel und Geruchsverbesserer. Salben, Pasten, Cremes und Gele können neben einer oder mehreren erfindungsgemäß verwendeten Verbindungen die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben einer oder mehreren erfindungsgemäß verwendeten Verbindungen die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether.

Lösungen und Emulsionen können neben einer oder mehreren erfindungsgemäß verwendeten Verbindungen die üblichen Trägerstoffe, wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöle, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe, enthalten.

Suspensionen können neben einer oder mehreren erfindungsgemäß verwendeten Verbindungen die üblichen Trägerstoffe, wie flüssige Verdünnungsmittel, z. B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe, enthalten.

Seifen können neben einer oder mehreren erfindungsgemäß verwendeten Verbindungen die üblichen Trägerstoffe, wie Alkalisalze von Fettsäuren, Salze von Fettsäurehalbestem, Fettsäureeiweißhydrolysaten, Isothionate, Lanolin, Fettalkohol, Pflanzenöle, Pflanzenextrakte, Glycerin, Zucker oder Gemische dieser Stoffe, enthalten.

Tensidhaltige Reinigungsprodukte können neben einer oder mehreren erfindungsgemäß verwendeten Verbindungen die üblichen Trägerstoffe, wie Salze von Fettalkoholsulfaten, Fettalkoholethersulfaten, Sulfobernsteinsäurehalbestern, Fettsäureeiweißhydrolysaten, Isothionaten, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe, enthalten.

Gesichts- und Körperöle können neben einer oder mehreren erfindungsgemäß verwendeten Verbindungen die üblichen Trägerstoffe, wie synthetische Öle, wie Fettsäureester, Fettalkohole, Silikonöle, natürliche Öle, wie Pflanzenöle und ölige Pflanzenauszüge, Paraffinöle, Lanolinöle oder Gemische dieser Stoffe, enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun- Präparate.

Mindestens eine erfindungsgemäß verwendete Verbindung liegt in der topischen Zusammensetzung in einer Menge von vorzugsweise 0,0001 bis 50 Gew.-%, besonders bevorzugt 0,001 bis 10 Gew.-%, insbesondere bevorzugt 0, 1 bis 1 Gew.-%, bezogen auf die Zusammensetzung, vor.

### Test zur Untersuchung der Radikalfängereigenschaften bzw. der antioxidativen Wirkung

Der Assay dient zum Screenen der antioxidativen oder Radikalfänger-Eigenschaft einer Substanz oder eines Extrakts. Um diese Eigenschaft zu ermitteln, läßt man die Substanz mit dem stabilen Radikal DPPH* (2,2-Diphenyl-1-picrylhydrazyl Hydrat) in ethanolischer Lösung reagieren. Die Reduktion von DPPH* wird über die Abnahme der Extinktion bei der charakteristischen Wellenlänge des Radikals verfolgt. In seiner radikalischen Form absorbiert DPPH* bei 515 nm, bei der Reduktion durch ein Antioxidans (AOX) nimmt die Extinktion ab. Für jedes Antioxidans werden unterschiedliche Konzentrationen untersucht (ausgedrückt als das Verhältnis von Mol Antioxidans / Mol DPPH*). Die Abnahme der Ektinktion bei 515 nm wird nach 1 Sekunde, 2 Minuten, 10 Minuten, und dann alle 10 Minuten bestimmt, bis die Extinktion konstant bleibt. Die exakte Anfangskonzentration von DPPH* wird mit Hilfe des Extinktionskoeffizienten bestimmt. Bei jeder Antioxidanskonzentration wird die verbliebene DPPH*-Konzentration als Prozent der Ausgangskonzentration ermittelt und gegen das molare Verhältnis von Antioxidans mit DPPH* aufgetragen. Die antiradikalische Aktivität wird definiert als der Anteil des Antioxidans, der die DPPH Konzentration auf 50 Prozent der Anfangsmenge senkt (Efficient Concentration = EC₅₀). Je kleiner dieser Wert ist, dest größer ist die Aktivität gegen Radikale. Das Reaktionsverhalten der einzelnen Antioxidantien ist sehr unterschiedlich. Man kann zwischen schnell-, mittel- und langsam reagierenden Substanzen unterscheiden, das Erreichen des steady-state kann zwischen 30 Sekunden und 12 Stunden liegen.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel:
Ethylacetat/Methanol 9:1.
- Massenspektrometrie (MS):: EI (Elektronenstoß-Ionisation) M⁺ FAB (Fast Atom Bombardment) (M+H)⁺ ESI (Electrospray lonization) (M+H)⁺ (wenn nichts anderes angegeben)

### Beispiel 1

### Herstellung von 6-Hydroxy-2-[3-(4-tert.-butylphenyl)-[1,2,4]-oxadiazol-5-yl]-chromon

1.1 3,25 g Natrium werden portionsweise unter Rühren in 300 ml Ethanol gelöst. Anschließend wird eine Lösung von 4,3 g 2,5-Dihydroxyacetophenon und 15,5 ml Oxalsäurediethylester in 25 ml Ethanol zugetropft. Danach wird das Reaktionsgemisch 3 Stunden auf 80° erhitzt. Man kühlt auf Raumtemperatur ab und tropft 10 ml 32 %ige HCI zu. Man erhitzt 30 Minuten bei 90°, kühlt ab, und entfernt das Lösungsmittel. Nach üblicher Aufarbeitung erhält man 5,9 g 6-Hydroxy-2-ethoxycarbonyl-chromon ("AA").
1.2 Eine Lösung von 2,0 g "AA" in 30 ml Essigsäure und 20 ml 32 %iger HCl wird unter Rückfluß erhitzt. Man kühlt ab, gießt auf Eis, filtriert ab und kristallisiert aus Ethanol um.
   Man erhält 1,5 g 6-Hydroxy-2-carbonsäure-chromon ("AB").
1.3 Zu einer Lösung von 150 mg "AB" in 10 ml THF gibt man unter Stickstoffatmosphäre und bei -10° 0,51 ml Triethylamin und 0,14 ml Isobutylchloroformiat. Man rührt eine Stunde nach und gibt danach eine Lösung von 280 mg 4-tert.-Butylbenzarnidoxim in THF zu. Man rührt 30 Minuten bei Raumtemperatur, 90 Minuten unter Rückfluß, arbeitet wie üblich auf und erhält 123 mg 6-Hydroxy-2-[3-(4-tert.-butylphenyl)-[1,2,4]-oxadiazol-5-yl]-chromon; EI MS (m/z) 362 (M⁺), 347 (M-Me⁺)
   UV-vis (ⁱPrOH): λₘₐₓ (Abs): 359 (0,12), 262.50 (1.02), 203.50 (1.08)

Analog erhält man die Verbindung 7-Hydroxy-2-[3-(4-tert.-butylphenyl)-[1,2,4]-oxadiazol-5-yl]-chromon.

### Beispiel 2

Analog Beispiel 1 erhält man durch Umsetzung von "AB" mit 2-Pyridinamidoxim die Verbindung 6-Hydroxy-2-[3-(pyridin-2-yl)-[1,2,4]-oxadiazol-5-yl]-chromon.

### Pharmakologischer Test

Die Verbindung 6-Hydroxy-2-[3-(4-tert.-butylphenyl)-[1,2,4]-oxadiazol-5-yl]-chromon ist ein schlechter PKB-Inhibitor im micromolaren Bereich (IC₅₀= >30µmol) und ein guter Tie2-Inhibitor (IC₅₀= >10µmol).

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen: Jede Ampulle enthält 10 mg Wirkstoff.

## Patentansprüche

1. Verbindungen der Formel I 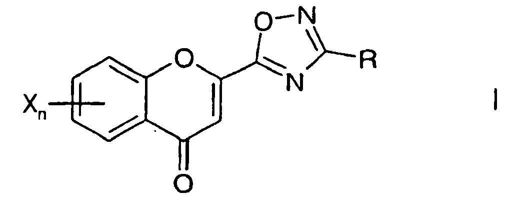
worin
R A, 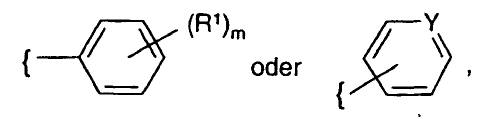
X H, -OH, -OA, Phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO oder SO₂NH₂,
zwei Reste X zusammen auch Methylendioxy oder Ethylendioxy,
R¹ H, A, -OH, -OA oder Hal,
zwei Reste R¹ zusammen auch Methylendioxy oder Ethylendioxy,
Y CH oder N,
Ar unsubstituiertes oder ein-, zwei- oder dreifach durch A substituiertes Phenyl,
A unverzweigtes oder verzweigtes Alkyl mit 1-10 C-Atomen, wobei 1-7 H-Atome durch F ersetzt sein können,
Hal F, Cl, Br oder I,
n 1, 2, 3 oder 4,
m 1, 2, 3, 4 oder 5
bedeuten,
sowie ihre pharmazeutisch verwendbaren Derivate, Solvate und Stereoisomere, einschließlich deren Mischungen in allen Verhältnissen.

2. Verbindungen nach Anspruch 1,
worin
X H, -OH, -OA,
zwei Reste X zusammen auch Methylendioxy oder Ethylendioxy, bedeutet,
sowie ihre pharmazeutisch verwendbaren Derivate, Solvate und Stereoisomere, einschließlich deren Mischungen in allen Verhältnissen.

3. Verbindungen nach Anspruch 1 oder 2,
worin
R A oder 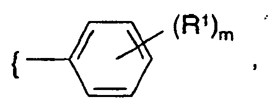
X H, -OH, -OA,
zwei Reste X zusammen auch Methylendioxy oder Ethylendioxy, bedeuten,
sowie ihre pharmazeutisch verwendbaren Derivate, Solvate und Stereoisomere, einschließlich deren Mischungen in allen Verhältnissen.

4. Verbindungen nach Anspruch 1 gemäß Anspruch 1,
worin
R A, 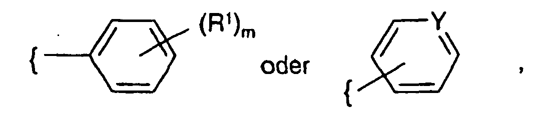
X H, -OH oder -OA,
Y N,
R¹ A,
A unverzweigtes oder verzweigtes Alkyl mit 1-6 C-Atomen,
n 1, 2, 3 oder 4,
m 1, 2, 3 oder 4,
bedeuten,
sowie ihre pharmazeutisch verwendbaren Derivate, Solvate und Stereoisomere, einschließlich deren Mischungen in allen Verhältnissen.

5. Verbindungen nach Anspruch 1 gemäß Anspruch 1,
worin
R 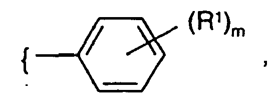
X H, -OH oder -OA,
R¹ A,
A unverzweigtes oder verzweigtes Alkyl mit 1-6 C-Atomen,
n 1, 2, 3 oder 4,
m 1, 2, 3 oder 4,
bedeuten,
sowie ihre pharmazeutisch verwendbaren Derivate, Solvate und Stereoisomere, einschließlich deren Mischungen in allen Verhältnissen.

6. Verbindungen gemäß Anspruch 1 ausgewählt aus der Gruppe
6-Hydroxy-2-[3-(4-tert.-butylphenyl)-[1,2,4]-oxadiazol-5-yl]-chromon,
7-Hydroxy-2-[3-(4-tert.-butylphenyl)-[1,2,4]-oxadiazol-5-yl]-chromon,
6-Hydroxy-2-[3-(pyridin-2-yl)-[1,2,4]-oxadiazol-5-yl]-chromon,
sowie ihre pharmazeutisch verwendbaren Derivate, Solvate und Stereoisomere, einschließlich deren Mischungen in allen Verhältnissen.

7. Verfahren zur Herstellung von Verbindungen der Formel 1 nach den Ansprüchen 1-6 sowie ihrer pharmazeutisch verwendbaren Derivate, Solvate und Stereoisomere, **dadurch gekennzeichnet, daß** man
a) zunächst eine Verbindung der Formel II 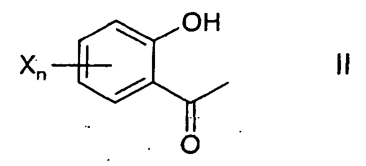
worin
X und n die in Anspruch 1 angegebene Bedeutung haben, mit einer Verbindung der Formel III 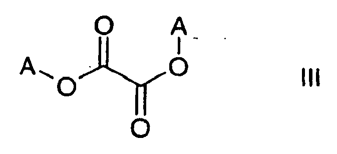
worin A Alkyl mit 1, 2, 3, 4, 5 oder 6 C-Atomen bedeutet, zu einer Verbindung der Formel IV 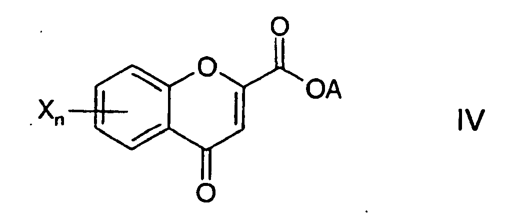
worin n die in Anspruch 1 angegebene Bedeutung hat und A Alkyl mit 1, 2, 3, 4, 5 oder 6 C-Atomen bedeutet, umsetzt,
b) anschließend den Ester IV zur Carbonsäure V hydrolysiert 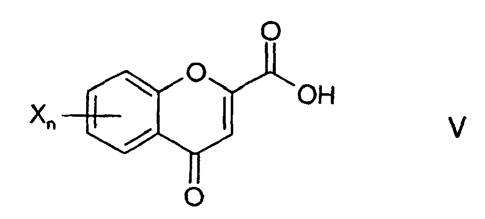
worin X und n die in Anspruch 1 angegebene Bedeutung haben,
c) anschließend die Carbonsäure V in das entsprechende Säurechlorid VI 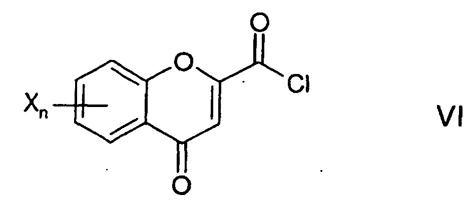
worin X und n die in Anspruch 1 angegebene Bedeutung haben, überführt,
und dann
entweder die Verbindung der Formel VI mit einer Verbindung der Formel VII 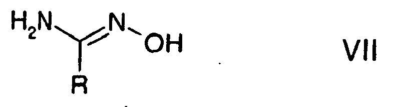
worin R die in Anspruch 1 angegebene Bedeutung hat, zu einer Verbindung der Formel I umsetzt,
oder die Verbindung der Formel V mit einer Verbindung der Formel VII,
worin R die in Anspruch 1 angegebene Bedeutung hat,
in einer zweistufigen Eintopf-Reaktion zu einer Verbindung der Formel I umsetzt, und/oder
d) eine Base oder Säure der Formel I in eines ihrer Salze umwandelt.

8. Verbindungen der Formel I nach einem oder mehreren der Ansprüche 1 bis 6 als Inhibitoren der Tyrosinkinase.

9. Arzneimittel, enthaltend mindestens eine Verbindung der Formel I nach einem oder mehreren der Ansprüche 1 bis 6 und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

10. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von festen Tumoren bei einem Säugetier, das einer derartigen Behandlung bedarf, wobei man dem Säugetier eine therapeutisch wirksame Menge einer Verbindung nach Anspruch 1 verabreicht.

11. Verwendung nach Anspruch 10, wobei der feste Tumor aus der Gruppe Gehirntumor, Tumor des Urogenitaltrakts, Tumor des lymphatischen Systems, Magentumor, Kehlkopftumor und Lungentumor stammt.

12. Verwendung nach Anspruch 10, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom stammt.

13. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung einer Krankheit, an der Angiogenese beteiligt ist bei einem Säugetier, das einer derartigen Behandlung bedarf, wobei man dem Säugetier, das einer derartigen Behandlung bedarf, eine therapeutisch wirksame Menge einer Verbindung nach Anspruch 1 verabreicht.

14. Verwendung nach Anspruch 13, wobei es sich bei der Krankheit um eine Augenkrankheit handelt.

15. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von Retina-Vaskularisierung bei einem Säugetier, das einer derartigen Behandlung bedarf, wobei man dem Säugetier, das einer derartigen Behandlung bedarf, eine therapeutisch wirksame Menge einer Verbindung nach Anspruch 1 verabreicht.

16. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von diabetischer Retinopathie bei einem Säugetier, das einer derartigen Behandlung bedarf, wobei man dem Säugetier, das einer derartigen Behandlung bedarf, eine therapeutisch wirksame Menge einer Verbindung nach Anspruch 1 verabreicht.

17. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von altersbedirigter Makula-Degeneration bei einem Säugetier, das einer derartigen Behandlung bedarf, wobei man dem Säugetier, das einer derartigen Behandlung bedarf, eine therapeutisch wirksame Menge einer Verbindung nach Anspruch 1 verabreicht.

18. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von Entzündungskrankheiten bei einem Säugetier, das einer derartigen Behandlung bedarf, wobei man dem Säugetier, das einer derartigen Behandlung bedarf, eine therapeutisch wirksame Menge einer Verbindung nach Anspruch 1 verabreicht.

19. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von Entzündungskrankheiten, wobei die Entzündungskrankheit aus der Gruppe rheumatoide Arthritis, Schuppenflechte, Kontaktdermatitis und Spät-Typ der Überempfindlichkeitsreaktion stammt.

20. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung einer tyrosinkinasebedingten Krankheit bzw. eines tyrosinkinasebedingten Leidens bei einem Säugetier, das einer derartigen Behandlung bedarf, wobei man dem Säugetier, das einer derartigen Behandlung bedarf, eine therapeutisch wirksame Menge einer Verbindung nach Anspruch 1 verabreicht.

21. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von Knochen-Pathologien bei einem Säugetier, das einer derartigen Behandlung bedarf, wobei die Knochenpathologie aus der Gruppe Osteosarkom, Osteoarthritis und Rachitis stammt, das **dadurch gekennzeichnet ist, dass** man eine therapeutisch wirksame Menge einer Verbindung nach Anspruch 1 verabreicht.

22. Arzneimittel enthaltend mindestens eine Verbindung der Formel I gemäß einem oder mehreren der Ansprüche 1 bis 6 und/oder ihre pharmazeutisch verwendbaren Derivate, Solvate und Stereoisomere, einschließlich deren Mischungen in allen Verhältnissen, und mindestens einen weiteren Arzneimittelwirkstoff.

23. Set (Kit), bestehend aus getrennten Packungen von
(a) einer wirksamen Menge an einer Verbindung der Formel I gemäß einem oder mehreren der Ansprüche 1 bis 6 und/oder ihrer pharmazeutisch verwendbaren Derivate, Solvate und Stereoisomere, einschließlich deren Mischungen in allen Verhältnissen, und
(b) einer wirksamen Menge eines weiteren Arzneimittelswirkstoffs.

24. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von festen Tumoren bei einem Säugetier, das einer derartigen Behandlung bedarf, **dadurch gekennzeichnet, dass** man eine therapeutisch wirksame Menge einer Verbindung nach Anspruch 1 in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht.

25. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von festen Tumoren bei einem Säugetier, das einer derartigen Behandlung bedarf, **dadurch gekennzeichnet, dass** man eine therapeutisch wirksame Menge einer Verbindung nach Anspruch 1 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht.

26. Verwendung von Verbindungen gemäß einem oder mehreren der Ansprüche 1 bis 6 und/oder ihre physiologisch unbedenklichen Salze und Solvate zur Herstellung eines Arzneimittels zur Behandlung von Krankheiten und/oder Fehlfunktionen, die durch oxidative Stressbedingungen charakterisiert sind, bei einem Säugetier, das einer derartigen Behandlung bedarf, wobei man dem Säugetier, das einer derartigen Behandlung bedarf, eine therapeutisch wirksame Menge einer Verbindung nach Anspruch 1 oral verabreicht.

27. Verwendung nach Anspruch 26, wobei es sich bei den Krankheiten und/oder Fehlfunktionen um Gedächtnisverlust und neurodegenerative Erkrankungen handelt.

28. Verwendung von Verbindungen gemäß einem oder mehreren der Ansprüche 1 bis 6 und/oder ihre physiologisch unbedenklichen Salze und Solvate als Nahrungsmittelzusatzstoffe.

29. Verwendung von Verbindungen gemäß einem oder mehreren der Ansprüche 1 bis 6 und/oder ihre physiologisch unbedenklichen Salze und Solvate in kosmetischen Formulierungen.

30. Verwendung nach Anspruch 29 zum Schutz der Streßproteine der Haut.

31. Verwendung nach Anspruch 29 oder 30 in Form einer topischen Zusammensetzung.

32. Verwendung nach Anspruch 29, 30 oder 31, **dadurch gekennzeichnet, daß** mindestens eine verwendete Verbindung in einer topischen Zusammensetzung in einer Menge von 0,0001 bis 50 Gew.-%, bezogen auf die Zusammensetzung, vorliegt.

## Claims

1. Compounds of the formula I 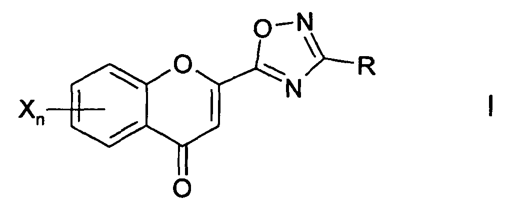
in which
R denotes A, 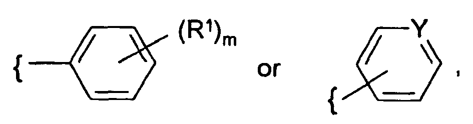
X denotes H, -OH, -OA, phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO or SO₂NH₂,
two radicals X together alternatively denote methylenedioxy or ethylenedioxy,
R¹ denotes H, A, -OH, -OA or Hal,
two radicals R¹ together alternatively denote methylenedioxy or ethylenedioxy,
Y denotes CH or N,
Ar denotes phenyl which is unsubstituted or mono-, di- or trisubstituted by A,
A denotes unbranched or branched alkyl having 1-10 C atoms, in which 1-7 H atoms may be replaced by F,
Hal denotes F, Cl, Br or I,
n denotes 1, 2, 3 or 4,
m denotes 1, 2, 3, 4 or 5,
and pharmaceutically usable derivatives, solvates and stereoisomers thereof, including mixtures thereof in all ratios.

2. Compounds according to Claim 1,
in which
X denotes H, -OH, -OA,
two radicals X together alternatively denote methylenedioxy or ethylenedioxy,
and pharmaceutically usable derivatives, solvates and stereoisomers thereof, including mixtures thereof in all ratios.

3. Compounds according to Claim 1 or 2,
in which
R denotes A or 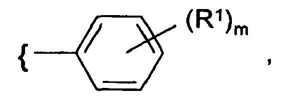
X denotes H, -OH, -OA,
two radicals X together alternatively denote methylenedioxy or ethylenedioxy,
and pharmaceutically usable derivatives, solvates and stereoisomers thereof, including mixtures thereof in all ratios.

4. Compounds according to Claim 1,
in which
R denotes A, 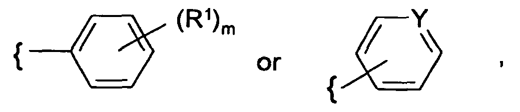
X denotes H, -OH or -OA,
Y denotes N,
R¹ denotes A,
A denotes unbranched or branched alkyl having 1-6 C atoms,
n denotes 1, 2, 3 or 4,
m denotes 1, 2, 3 or 4,
and pharmaceutically usable derivatives, solvates and stereoisomers thereof, including mixtures thereof in all ratios.

5. Compounds according to Claim 1,
in which
R denotes 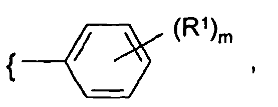
X denotes H, -OH or -OA,
R¹ denotes A,
A denotes unbranched or branched alkyl having 1-6 C atoms,
n denotes 1, 2, 3 or 4,
m denotes 1, 2, 3 or 4,
and pharmaceutically usable derivatives, solvates and stereoisomers thereof, including mixtures thereof in all ratios.

6. Compounds according to Claim 1 selected from the group consisting of
6-hydroxy-2-[3-(4-tert-butylphenyl)-1,2,4-oxadiazol-5-yl]chromone, 7-hydroxy-2-[3-(4-tert-butylphenyl)-1,2,4-oxadiazol-5-yl]chromone, 6-hydroxy-2-[3-(pyridin-2-yl)-1,2,4-oxadiazol-5-yl]chromone,
and pharmaceutically usable derivatives, solvates and stereoisomers thereof, including mixtures thereof in all ratios.

7. Process for the preparation of compounds of the formula I according to Claims 1-6 and pharmaceutically usable derivatives, solvates and stereoisomers thereof, **characterised in that**
a) firstly a compound of the formula II 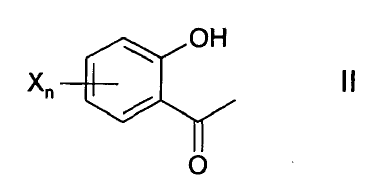
in which
X and n have the meaning indicated in Claim 1, is reacted with a compound of the formula III 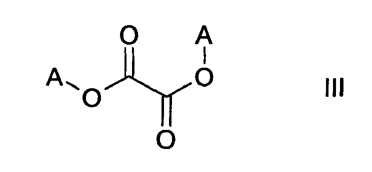
in which A denotes alkyl having 1, 2, 3, 4, 5 or 6 C atoms, to give a compound of the formula IV 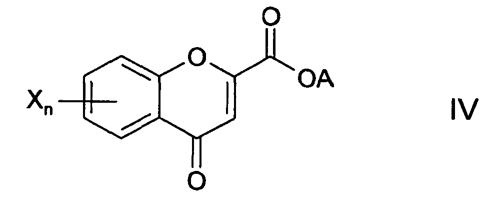
in which n has the meaning indicated in Claim 1 and A denotes alkyl having 1, 2, 3, 4, 5 or 6 C atoms,
b) subsequently the ester IV is hydrolysed to the carboxylic acid V 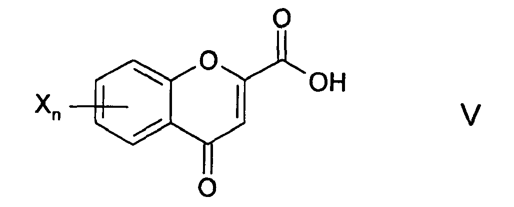
in which X and n have the meaning indicated in Claim 1,
c) subsequently the carboxylic acid V is converted into the corresponding acid chloride VI 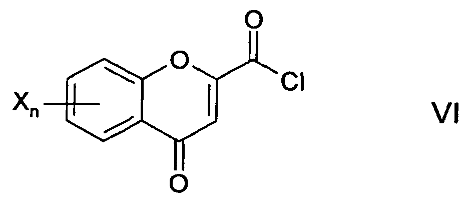 in which X and n have the meaning indicated in Claim 1,
and then
either the compound of the formula VI is reacted with a compound of the formula VII 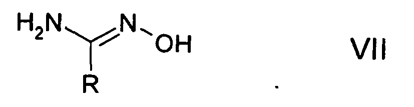
in which R has the meaning indicated in Claim 1, to give a compound of the formula I,
or the compound of the formula V is reacted with a compound of the formula VII,
in which R has the meaning indicated in Claim 1,
in a two-step, one-pot reaction to give a compound of the formula I, and/or
d) a base or acid of the formula I is converted into one of its salts.

8. Compounds of the formula I according to one or more of Claims 1 to 6 as inhibitors of tyrosine kinase.

9. Medicaments comprising at least one compound of the formula I according to one or more of Claims 1 to 6 and/or pharmaceutically usable derivatives, solvates and stereoisomers thereof, including mixtures thereof in all ratios, and optionally excipients and/or adjuvants.

10. Use of compounds according to one or more of Claims 1 to 6 and/or physiologically acceptable salts and solvates thereof for the preparation of a medicament for the treatment of solid tumours in a mammal in need of such treatment, in which a therapeutically effective amount of a compound according to Claim 1 is administered to the mammal.

11. Use according to Claim 10, where the solid tumour originates from the group of cerebral tumour, tumour of the genito-urinary tract, tumour of the lymphatic system, stomach tumour, laryngeal tumour and lung tumour.

12. Use according to Claim 10, where the solid tumour originates from the group of monocytic leukaemia, lung adenocarcinoma, small cell lung carcinomas, pancreatic cancer, glioblastomas and breast carcinoma.

13. Use of compounds according to one or more of Claims 1 to 6 and/or physiologically acceptable salts and solvates thereof for the preparation of a medicament for the treatment of a disease in which angiogenesis is implicated in a mammal in need of such treatment, in which a therapeutically effective amount of a compound according to Claim 1 is administered to the mammal in need of such treatment.

14. Use according to Claim 13, where the disease is an ocular disease.

15. Use of compounds according to one or more of Claims 1 to 6 and/or physiologically acceptable salts and solvates thereof for the preparation of a medicament for the treatment of retinal vascularisation in a mammal in need of such treatment, in which a therapeutically effective amount of a compound according to Claim 1 is administered to the mammal in need of such treatment.

16. Use of compounds according to one or more of Claims 1 to 6 and/or physiologically acceptable salts and solvates thereof for the preparation of a medicament for the treatment of diabetic retinopathy in a mammal in need of such treatment, in which a therapeutically effective amount of a compound according to Claim 1 is administered to the mammal in need of such treatment.

17. Use of compounds according to one or more of Claims 1 to 6 and/or physiologically acceptable salts and solvates thereof for the preparation of a medicament for the treatment of age-related macular degeneration in a mammal in need of such treatment, in which a therapeutically effective amount of a compound according to Claim 1 is administered to the mammal in need of such treatment.

18. Use of compounds according to one or more of Claims 1 to 6 and/or physiologically acceptable salts and solvates thereof for the preparation of a medicament for the treatment of inflammatory diseases in a mammal in need of such treatment, in which a therapeutically effective amount of a compound according to Claim 1 is administered to the mammal in need of such treatment.

19. Use of compounds according to one or more of Claims 1 to 6 and/or physiologically acceptable salts and solvates thereof for the preparation of a medicament for the treatment of inflammatory diseases, where the inflammatory disease originates from the group of rheumatoid arthritis, psoriasis, contact dermatitis and delayed hypersensitivity reaction.

20. Use of compounds according to one or more of Claims 1 to 6 and/or physiologically acceptable salts and solvates thereof for the preparation of a medicament for the treatment of a tyrosine kinase-dependent disease or a tyrosine kinase-dependent condition in a mammal in need of such treatment, in which a therapeutically effective amount of a compound according to Claim 1 is administered to the mammal in need of such treatment.

21. Use of compounds according to one or more of Claims 1 to 6 and/or physiologically acceptable salts and solvates thereof for the preparation of a medicament for the treatment of bone pathologies in a mammal in need of such treatment, where the bone pathology originates from the group of osteosarcoma, osteoarthritis and rickets, which is **characterised in that** a therapeutically effective amount of a compound according to Claim 1 is administered.

22. Medicaments comprising at least one compound of the formula I according to one or more of Claims 1 to 6 and/or pharmaceutically usable derivatives, solvates and stereoisomers thereof, including mixtures thereof in all ratios, and at least one further medicament active ingredient.

23. Set (kit) consisting of separate packs of
(a) an effective amount of a compound of the formula I according to one or more of Claims 1 to 6 and/or pharmaceutically usable derivatives, solvates and stereoisomers thereof, including mixtures thereof in all ratios, and
(b) an effective amount of a further medicament active ingredient.

24. Use of compounds according to one or more of Claims 1 to 6 and/or physiologically acceptable salts and solvates thereof for the preparation of a medicament for the treatment of solid tumours in a mammal in need of such treatment, **characterised in that** a therapeutically effective amount of a compound according to Claim 1 is administered in combination with a compound from the group of 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

25. Use of compounds according to one or more of Claims 1 to 6 and/or physiologically acceptable salts and solvates thereof for the preparation of a medicament for the treatment of solid tumours in a mammal in need of such treatment, **characterised in that** a therapeutically effective amount of a compound according to Claim 1 is administered in combination with radiotherapy and a compound from the group of 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

26. Use of compounds according to one or more of Claims 1 to 6 and/or physiologically acceptable salts and solvates thereof for the preparation of a medicament for the treatment of diseases and/or dysfunctions which are **characterised by** oxidative stress conditions in a mammal in need of such treatment, in which a therapeutically effective amount of a compound according to Claim 1 is administered orally to the mammal in need of such treatment.

27. Use according to Claim 26, where the diseases and/or dysfunctions are memory loss and neurodegenerative diseases.

28. Use of compounds according to one or more of Claims 1 to 6 and/or physiologically acceptable salts and solvates thereof as food supplements.

29. Use of compounds according to one or more of Claims 1 to 6 and/or physiologically acceptable salts and solvates thereof in cosmetic formulations.

30. Use according to Claim 29 for protection of the stress proteins of the skin.

31. Use according to Claim 29 or 30 in the form of a topical composition.

32. Use according to Claim 29, 30 or 31, **characterised in that** at least one compound used is present in a topical composition in an amount of 0.0001 to 50% by weight, based on the composition.

## Revendications

1. Composés de la formule I 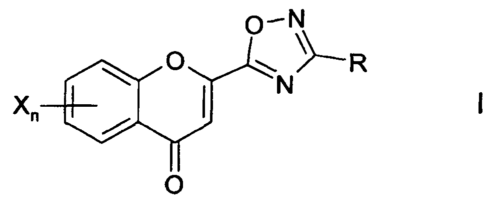
dans laquelle
R représente A, 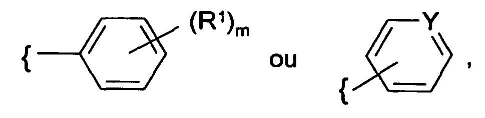
X représente H, -OH, -OA, phénoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO ou SO₂NH₂,
deux radicaux X représentent ensemble à titre d'alternative méthylènedioxy ou éthylènedioxy,
R¹ représente H, A, -OH, -OA ou Hal,
deux radicaux R¹ représentent ensemble à titre d'alternative méthylènedioxy ou éthylènedioxy,
Y représente CH ou N,
Ar représente phényle qui est non substitué ou mono-, di- ou trisubstitué par A,
A représente un alkyle non ramifié ou ramifié comportant de 1 à 10 atomes de C, où 1 à 7 atomes de H peuvent être remplacés par F,
Hal représente F, Cl, Br ou I,
n représente 1, 2, 3 ou 4,
m représente 1, 2, 3, 4 ou 5,
et leurs dérivés, leurs solvates et leurs stéréoisomères utilisables pharmaceutiquement, incluant leurs mélanges selon tous les rapports.

2. Composés selon la revendication 1,
selon lesquels
X représente H, -OH, -OA,
deux radicaux X représentent ensemble à titre d'alternative méthylènedioxy ou éthylènedioxy,
et leurs dérivés, leurs solvates et leurs stéréoisomères utilisables pharmaceutiquement, incluant leurs mélanges selon tous les rapports.

3. Composés selon la revendication 1 ou 2, selon lesquels
R représente A ou 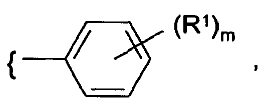
X représente H, -OH, -OA,
deux radicaux X représentent ensemble à titre d'alternative méthylènedioxy ou éthylènedioxy,
et leurs dérivés, leurs solvates et leurs stéréoisomères utilisables pharmaceutiquement, incluant leurs mélanges selon tous les rapports.

4. Composés selon la revendication 1, selon lesquels
R représente A 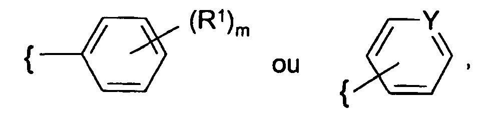
X représente H, -OH ou -OA,
Y représente N,
R¹ représente A,
A représente un alkyle non ramifié ou ramifié comportant de 1 à 6 atomes de C,
n représente 1, 2, 3 ou 4,
m représente 1, 2, 3 ou 4,
et leurs dérives, leurs solvates et leurs stéréoisomères utilisables pharmaceutiquement, incluant leurs mélanges selon tous les rapports.

5. Composés selon la revendication 1, selon lesquels
R représente 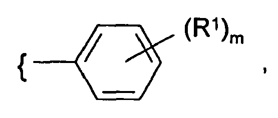
X représente H, -OH ou -OA,
R¹ représente A,
A représente un alkyle non ramifié ou ramifié comportant de 1 à 6 atomes de C,
n représente 1, 2, 3 ou 4,
m représente 1, 2, 3 ou 4,
et leurs dérives, leurs solvates et leurs stéréoisomères utilisables pharmaceutiquement, incluant leurs mélanges selon tous les rapports.

6. Composés selon la revendication 1 choisis parmi le groupe constitué par
6-hydroxy-2-[3-(4-tert-butylphényl)-1,2,4-oxadiazol-5-yl]chromone, 7-hydroxy-2-[3-(4-tert-butylphényl)-1,2,4-oxadiazol-5-yl]chromone, 6-hydroxy-2-[3-(pyridine-2-yl)-1,2,4-oxadiazol-5-yl]chromone,
et leurs dérives, leurs solvates et leurs stéréoisomères utilisables pharmaceutiquement, incluant leurs mélanges selon tous les rapports.

7. Procédé pour la préparation de composés de la formule I selon les revendications 1 à 6 et de leurs dérivés, de leurs solvates et de leurs stéréoisomères utilisables pharmaceutiquement, **caractérisé en ce que**
a) tout d'abord, un composé de la formule II 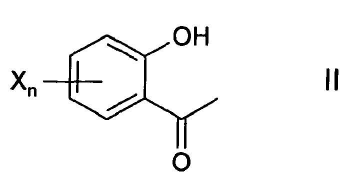
dans laquelle
X et n présentent la signification indiquée selon la revendication 1, est amené à réagir avec un composé de la formule III 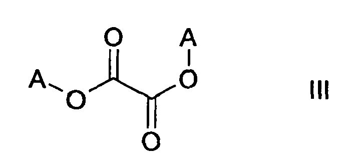
dans laquelle A représente alkyle comportant 1, 2, 3, 4, 5 ou 6 atomes de C,
afin d'obtenir un composé de la formule IV 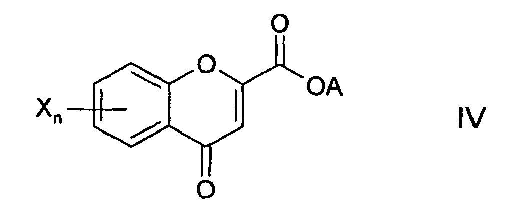
dans laquelle n présente la signification indiquée selon la revendication 1,
et A représente alkyle comportant 1, 2, 3, 4, 5 ou 6 atomes de C,
b) ensuite, l'ester IV est hydrolysé selon l'acide carboxylique V 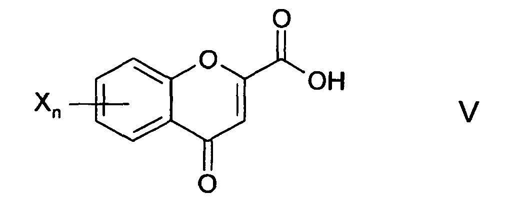 selon lequel X et n présentent la signification indiquée selon la revendication 1,
c) ensuite, l'acide carboxylique V est converti selon le chlorure d'acide correspondant VI 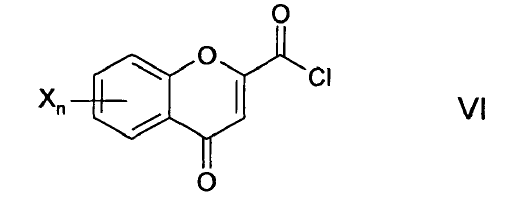 selon lequel X et n présentent la signification indiquée selon la revendication 1,
puis
soit le composé de la formule VI est amené à réagir avec un composé de la formule VII 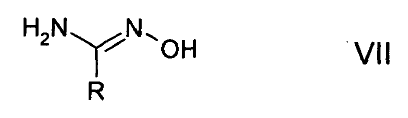
dans laquelle R présente la signification indiquée selon la revendication 1,
pour donner un composé de la formule 1,
soit le composé de la formule V est amené à réagir avec un composé de la formule VII,
dans laquelle R présente la signification indiquée selon la revendication 1,
selon une réaction monocellulaire à deux étapes pour obtenir un composé de la formule I,
et/ou
d) une base ou un acide de la formule I est converti selon l'un de ses sels.

8. Composés de la formule I selon une ou plusieurs des revendications 1 à 6 en tant qu'inhibiteurs de tyrosine kinase.

9. Médicaments comprenant au moins un composé de la formule I selon une ou plusieurs des revendications 1 à 6 et/ou leurs dérivés, leurs solvates et leurs stéréoisomères utilisables pharmaceutiquement, incluant leurs mélanges selon tous les rapports et en option, des excipients et/ou des adjuvants.

10. Utilisation de composés selon une ou plusieurs des revendications 1 à 6 et/ou de leurs sels et de leurs solvates acceptables physiologiquement pour la préparation d'un médicament pour le traitement de tumeurs solides dans un mammifère ayant besoin d'un tel traitement où une quantité efficace thérapeutiquement d'un composé selon la revendication 1 est administrée au mammifère.

11. Utilisation selon la revendication 10, où la tumeur solide prend son origine parmi le groupe constitué par une tumeur cérébrale, une tumeur de l'appareil uro-génital, une tumeur du système lymphatique, une tumeur de l'estomac, une tumeur du larynx et une tumeur du poumon.

12. Utilisation selon la revendication 10, où la tumeur solide prend son origine parmi le groupe comprenant la leucémie monocytique, l'adénocarcinome du poumon, les carcinomes de petites cellules du poumon, le cancer du pancréas, les glioblastomes et le carcinome du sein.

13. Utilisation de composés selon une ou plusieurs des revendications 1 à 6 et/ou de leurs sels et de leurs solvates acceptables physiologiquement pour la préparation d'un médicament pour le traitement d'une maladie au niveau de laquelle une angiogénèse est impliquée au niveau d'un mammifère ayant besoin d'un tel traitement, où une quantité efficace thérapeutiquement d'un composé selon la revendication 1 est administrée au mammifère ayant besoin d'un tel traitement.

14. Utilisation selon la revendication 13, où la maladie est une maladie des yeux.

15. Utilisation de composés selon une ou plusieurs des revendications 1 à 6 et /ou de leurs sels et de leurs solvates acceptables physiologiquement pour la préparation d'un médicament pour le traitement d'une vascularisation de la rétine au niveau d'un mammifère ayant besoin d'un tel traitement, où une quantité efficace thérapeutiquement d'un composé selon la revendication 1 est administrée au mammifère ayant besoin d'un tel traitement.

16. Utilisation de composés selon une ou plusieurs des revendications 1 à 6 et/ou de leurs sels et de leurs solvates acceptables physiologiquement pour la préparation d'un médicament pour le traitement d'une rétinopathie diabétique au niveau d'un mammifère ayant besoin d'un tel traitement, où une quantité efficace thérapeutiquement d'un composé selon la revendication 1 est administrée au mammifère ayant besoin d'un tel traitement.

17. Utilisation de composés selon une ou plusieurs des revendications 1 à 6 et/ou de leurs sels et de leurs solvates acceptables physiologiquement pour la préparation d'un médicament pour le traitement d'une dégénérescence maculaire liée à l'âge au niveau d'un mammifère ayant besoin d'un tel traitement, où une quantité efficace thérapeutiquement d'un composé selon la revendication 1 est administrée au mammifère ayant besoin d'un tel traitement.

18. Utilisation de composés selon une ou plusieurs des revendications 1 à 6 et/ou de leurs sels et de leurs solvates acceptables physiologiquement pour la préparation d'un médicament pour le traitement de maladies inflammatoires au niveau d'un mammifère ayant besoin d'un tel traitement, où une quantité efficace thérapeutiquement d'un composé selon la revendication 1 est administrée au mammifère ayant besoin d'un tel traitement.

19. Utilisation de composés selon une ou plusieurs des revendications 1 à 6 et/ou de leurs sels et de leurs solvates acceptables physiologiquement pour la préparation d'un médicament pour le traitement de maladies inflammatoires, où la maladie inflammatoire prend son origine parmi le groupe constitué par l'arthrite rhumatoïde, le psoriasis, une dermatite par contact et une réaction d'hypersensibilité retardée.

20. Utilisation de composés selon une ou plusieurs des revendications 1 à 6 et/ou de leurs sels et de leurs solvates acceptables physiologiquement pour la préparation d'un médicament pour le traitement d'une maladie dépendant de la tyrosine kinase ou d'une condition dépendant de la tyrosine kinase au niveau d'un mammifère ayant besoin d'un tel traitement, où une quantité efficace thérapeutiquement d'un composé selon la revendication 1 est administrée au mammifère ayant besoin d'un tel traitement.

21. Utilisation de composés selon une ou plusieurs des revendications 1 à 6 et/ou de leurs sels et de leurs solvates acceptables physiologiquement pour la préparation d'un médicament pour le traitement de pathologies des os au niveau d'un mammifère ayant besoin d'un tel traitement, où la pathologie des os prend son origine parmi le groupe comprenant ostéosarcome, arthrose et rachitismes, qui est **caractérisée en ce qu'**une quantité efficace thérapeutiquement d'un composé selon la revendication 1 est administrée.

22. Médicaments comprenant au moins un composé de la formule I selon une ou plusieurs des revendications 1 à 6 et/ou leurs dérivés, leurs solvates et leurs stéréoisomères utilisables pharmaceutiquement, incluant leurs mélanges selon tous les rapports, et au moins un autre ingrédient actif de médicament.

23. Jeu (kit) constitué par des modules séparés de
(a) une quantité efficace d'un composé de la formule 1 selon une ou plusieurs des revendications 1 à 6 et/ou de leurs dérivés, de leurs solvates et de leurs stéréoisomères utilisables pharmaceutiquement incluant leurs mélanges selon tous les rapports,
et
(b) une quantité efficace d'un autre ingrédient actif de médicament.

24. Utilisation de composés selon une ou plusieurs des revendications 1 à 6 et/ou de leurs sels et de leurs solvates acceptables physiologiquement pour la préparation d'un médicament pour le traitement de tumeurs solides au niveau d'un mammifère ayant besoin d'un tel traitement, **caractérisée en ce qu'**une quantité efficace thérapeutiquement d'un composé selon la revendication 1 est administrée en combinaison avec un composé pris parmi le groupe constitué par 1) un modulateur de récepteur d'oestrogène, 2) un modulateur de récepteur d'androgène, 3) un modulateur de récepteur de rétinoïde, 4) un agent cytotoxique, 5) un agent d'antiprolifération, 6) un inhibiteur de transférase prényl-protéine, 7) un inhibiteur de réductase HMG-CoA, 8) un inhibiteur de protéase HIV, 9) un inhibiteur de transcriptase inverse et 10) un autre inhibiteur d'angiogénèse.

25. Utilisation de composés selon une ou plusieurs des revendications 1 à 6 et/ou de leurs sels et de leurs solvates acceptables physiologiquement pour la préparation d'un médicament pour le traitement de tumeurs solides au niveau d'un mammifère ayant besoin d'un tel traitement, **caractérisée en ce qu'**une quantité efficace thérapeutiquement d'un composé selon la revendication 1 est administrée en combinaison avec une radiothérapie et un composé pris parmi le groupe constitué par 1) un modulateur de récepteur d'oestrogène, 2) un modulateur de récepteur d'androgène, 3) un modulateur de récepteur de rétinoïde, 4) un agent cytotoxique, 5) un agent d'antiprolifération, 6) un inhibiteur de transférase prényl-protéine, 7) un inhibiteur de réductase HMG-CoA, 8) un inhibiteur de protéase HIV, 9) un inhibiteur de transcriptase inverse et 10) un autre inhibiteur d'angiogénèse.

26. Utilisation de composés selon une ou plusieurs des revendications 1 à 6 et/ou de leurs sels et de leurs solvates acceptables physiologiquement pour la préparation d'un médicament pour le traitement de maladies et/ou de dysfonctionnements qui sont **caractérisés par** des conditions de stress oxydatif au niveau d'un mammifère ayant besoin d'un tel traitement, où une quantité efficace thérapeutiquement d'un composé selon la revendication 1 est administrée par voie orale au mammifère ayant besoin d'un tel traitement.

27. Utilisation selon la revendication 26, où les maladies et/ou les dysfonctionnements sont des maladies de perte de mémoire et neuro-dégénératives.

28. Utilisation de composés selon une ou plusieurs des revendications 1 à 6 et/ou de leurs sels et de leurs solvates acceptables physiologiquement en tant que compléments alimentaires.

29. Utilisation de composés selon une ou plusieurs des revendications 1 à 6 et/ou de leurs sels et de leurs solvates acceptables physiologiquement au niveau de formulations cosmétiques.

30. Utilisation selon la revendication 29 pour la protection des protéines de contrainte de la peau.

31. Utilisation selon la revendication 29 ou 30 sous la forme d'une composition topique.

32. Utilisation selon la revendication 29, 30 ou 31, **caractérisée en ce qu'**au moins un composé utilisé est présent dans une composition topique selon une quantité de 0,0001 à 50% en poids, sur la base de la composition.
